# EUROPÄISCHE PATENTSCHRIFT

(11) **EP 2 364 989 B1**
(45) Veröffentlichungstag und Bekanntmachung des Hinweises auf die Patenterteilung: **05.03.2014**
(21) Anmeldenummer: 11167476.8
(22) Anmeldetag: 28.07.2006
(51) Int. Cl.: C07H 19/207, C07H 23/00, C07H 21/02, C07H 19/213, C07H 21/00, C12Q 1/32

(54) **Stabile NAD/NADH-Derivate**
Stable NAD/NADH-derivatives
Dérivés de NAD/NADH stables

(30) Priorität: 28.07.2005 DE 102005035461
(43) Veröffentlichungstag der Anmeldung: 14.09.2011
(62) Teilanmeldung aus: 06776486.0
(73) Patentinhaber: F. Hoffmann-La Roche AG, 4070 Basel (CH); Roche Diagnostics GmbH, 68305 Mannheim (DE)
(72) Erfinder: Hoenes, Joachim, 64673, Zwingenberg (DE); Heindl, Dieter, 82396, Pähl (DE); Horn, Carina, 68647, Biblis (DE); Gaessler-Dietsche, Claudia, 69198, Schriesheim (DE)
(74) Vertreter: Weiss, Wolfgang

(56) Entgegenhaltungen:
- US-A- 5 801 006
- SLAMA J T ET AL: "CARBANICOTINAMIDE ADENINE DINUCLEOTIDE SYNTHESIS AND ENZYMOLOGICAL PROPERTIES OF A CARBOCYCLIC ANALOGUE OF OXIDIZED NAD", BIOCHEMISTRY, Bd. 27, Nr. 1, 1988, Seiten 183-193, XP002405653, ISSN: 0006-2960
- HUTCHINSON, EDWARD J. ET AL: "Synthesis of carbocyclic NAD+ containing a methylenebisphosphonate linkage for the investigation of ADP-ribosyl cyclase", CHEMICAL COMMUNICATIONS (CAMBRIDGE) , (24), 2765-2766 CODEN: CHCOFS; ISSN: 1359-7345, 1996, XP008070860,
- ANDERSON B M: "ANALOGS OF PYRIDINE NUCLEOTIDE COENZYMES", PYRIDINE NUCLEOTIDE COENZYMES, 1982, Seiten 91-133, XP001085208,
- MULLER-STEFFNER, HELENE ET AL: "Photodependent inhibition of bovine spleen NAD+ glycohydrolase by 8-azido carbocyclic analogs of NAD+", BIOCHEMICAL AND BIOPHYSICAL RESEARCH COMMUNICATIONS , 228(1), 128-133 CODEN: BBRCA9; ISSN: 0006-291X, 1996, XP002405611,
- SLEATH P R ET AL: "PYRIDINE COENZYME ANALOGUES 3. SYNTHESIS OF THREE REDUCED NAD ANALOGUES CONTAINING A 2' DEOXY-2'-SUBSTITUTED NICOTINAMIDE ARABINOFURANOSYL MOIETY", JOURNAL OF ORGANIC CHEMISTRY, Bd. 56, Nr. 11, 1991, Seiten 3608-3613, XP002405612, ISSN: 0022-3263

## Beschreibung

Die Erfindung betrifft Testelemente umfassend stabile Nicotinamid-Adenin-Dinukleotid (NAD/NADH)-bzw. Nicotinamid-Adenin-Dinukleotidphosphat (NADP/NADPH)-Derivate und ihre Verwendung in biochemischen Nachweisverfahren.

Messsysteme zur biochemischen Analytik sind wichtige Bestandteile klinisch relevanter Analyseverfahren. Hierbei steht die Messung von Analyten, z.B. Metaboliten oder Substraten, im Vordergrund, welche mit Hilfe eines Enzyms direkt oder indirekt bestimmt werden. Die Analyten werden hierbei mit Hilfe eines Enzym-Coenzym-Komplexes umgesetzt und anschließend quantifiziert. Dabei wird der zu bestimmende Analyt mit einem geeigneten Enzym und einem Coenzym in Kontakt gebracht, wobei das Enzym meist in katalytischen Mengen eingesetzt wird. Das Coenzym wird durch die enzymatische Reaktion verändert, z.B. oxidiert bzw. reduziert. Dieser Vorgang kann direkt oder durch einen Mediator elektrochemisch oder photometrisch erfasst werden. Eine Kalibrierung liefert einen direkten Zusammenhang des Messwerts mit der Konzentration des zu bestimmenden Analyten.

Coenzyme sind organische Moleküle, die kovalent oder nicht-kovalent an ein Enzym gebunden sind und durch die Umsetzung des Analyten verändert werden. Prominente Beispiele für Coenzyme sind Nicotinamid-Adenin-Dinukleotid (NAD) bzw. Nicotinamid-Adenin-Dinukleotidphosphat (NADP), aus denen durch Reduktion NADH bzw. NADPH entstehen.

Aus dem Stand der Technik bekannte Messsysteme zeichnen sich durch eine zeitlich begrenzte Haltbarkeit sowie durch spezielle Anforderungen an die Umgebung, wie Kühlung oder trockene Lagerung, zur Erzielung dieser Haltbarkeit aus. Bei bestimmten Anwendungsformen, z.B. bei Tests, die vom Endverbraucher selbst durchgeführt werden, wie etwa beim Blutglucose-Selbstmonitoring, können daher Fehlergebnisse durch falsche, unbemerkte Fehllagerung vorkommen. Besonders die Erschöpfung von Trocknungsmitteln durch zu langes Öffnen der Primärverpackungen kann zu Fehlmessungen führen, die bei manchen Systemen vom Verbraucher kaum zu erkennen sind.

Eine bekannte Maßnahme, die zur Erhöhung der Stabilität von biochemischen Messsystemen eingesetzt wird, ist die Verwendung stabiler Enzyme, z.B. die Verwendung von Enzymen aus thermophilen Organismen. Weiterhin besteht die Möglichkeit, Enzyme durch chemische Modifizierung, z.B. Quervernetzung, oder durch Mutagenese zu stabilisieren. Darüber hinaus können auch Enzymstabilisatoren, wie z.B. Trehalose, Polyvinylpyrrolidon und Serumalbumin, zugesetzt werden oder die Enzyme z.B. durch Photopolymerisation in Polymernetzwerke eingeschlossen werden.

Weiterhin wird versucht, die Haltbarkeit biochemischer Messsysteme durch Verwendung stabiler Mediatoren zu verbessern. So wird durch die Verwendung von Mediatoren mit möglichst niedrigem Redoxpotential die Spezifität von Tests erhöht und Störungen während der Reaktion eliminiert. Eine untere Grenze für das Redoxpotential von Mediatoren bilden jedoch die Redoxpotentiale der Enzym/Coenzymkomplexe. Werden diese unterschritten, wird die Reaktion mit den Mediatoren verlangsamt oder gar unterbunden.

Alternativ können auch biochemische Messsysteme ohne Mediatoren verwendet werden, bei denen beispielsweise eine direkte Detektion von Coenzymen, z.B. des Coenzyms NADH, erfolgt. Ein Nachteil solcher Messsysteme besteht jedoch darin, dass Coenzyme, wie NAD und NADP, instabil sind.

NAD und NADP sind basenlabile Moleküle, deren Abbauwege in der Literatur beschrieben sind (N.J. Oppenheimer in The Pyridine Nucleotide Coenzyms Academic Press New York, London 1982, Hrsg. J. Everese, B. Anderson, K. You, Kapitel 3, Seiten 56-65). Im wesentlichen entsteht beim Abbau von NAD bzw. NADP ADP-Ribose, indem die Glycosyl-Bindungen zwischen der Ribose und der Pyridineinheit gespalten wird. Die reduzierten Formen NADH und NADPH sind hingegen säurelabil: z.B. ist die Epimerisierung ein bekannter Abbauweg. In beiden Fällen liegt der Instabilität von NAD/NADP und NADH/NADPH die Labilität der Glykosyl-Bindung zwischen der Ribose- und der Pyridineinheit zugrunde. Aber auch unter nicht drastischen Bedingungen, wie z.B. in wässriger Lösung, geschieht die Hydrolyse der Coenzyme NAD bzw. NADP allein schon durch die Umgebungsfeuchte. Diese Instabilität kann zu Ungenauigkeiten bei der Messung von Analyten führen.

Eine Reihe von NAD/NADP-Derivaten wird z.B. in B.M. Anderson in the Pyridine Nucleotide Coenzymes, Academic Press New York, London 1982 , Hrsg. J. Everese, B. Anderson, K. You, Kapitel 4 beschrieben. Die meisten dieser Derivate werden allerdings nicht gut von Enzymen akzeptiert. Das einzige Derivat, das daher bisher für diagnostische Tests verwendet wurde, ist 3-Acetylpyridin-Adenin-Dinukleotid (Acetyl NAD), weiches erstmals 1956 beschrieben wurde (N.O. Kaplan, J. Biol. Chem. (1956), 221, 823). Auch dieses Coenzym zeigt eine geringe Akzeptanz von Enzymen und eine Änderung im Redoxpotential.

In WO 01/94370 ist die Verwendung weiterer NAD-Derivate mit einer modifizierten Pyridin-Gruppe beschrieben. Modifikationen der Nicotinamid-Gruppe haben jedoch generell direkten Einfluss auf die katalytische Reaktion. In den meisten Fällen ist dieser Einfluss negativ.

In einem weiteren Stabilisierungskonzept wurde die Ribose-Einheit verändert, um dadurch die Stabilität der Glycosyl-Bindung zu beeinflussen. Dieses Vorgehen interferiert nicht direkt mit der katalytischen Reaktion der Nicotinamid-Gruppe. Es kann jedoch ein indirekter Einfluss bestehen, sobald das Enzym eine starke und spezifische Bindung an die Ribose-Einheit aufweist. Kaufmann et al. offenbaren diesbezüglich in WO 98/33936 und US 5,801,006 bzw. in WO 01/49247 eine Reihe von Thioribose-NAD Derivaten. Ein Zusammenhang zwischen der Modifizierung der Nicotinamid-Riboseeinheit und der Aktivität der Derivate in enzymatischen Reaktionen wurde bisher jedoch nicht gezeigt.

carbaNAD, ein Derivat ohne Glycosyl-Bindung, wurde erstmals 1988 beschrieben (J.T. Slama, Biochemistry 1989, 27, 183 und Biochemistry 1989, 28, 7688). Die Ribose wird hierin durch eine carbazyklische Zucker-Einheit substituiert. carbaNAD wurde zwar als Substrat für Dehydrogenasen beschrieben, seine Aktivität wurde bisher jedoch nicht in biochemischen Nachweisverfahren in der Klinik nachgewiesen.

Ein ähnlicher Ansatz wurde später von G.M. Blackburn, Chem. Comm., 1996, 2765 beschrieben, um carbaNAD mit einer Methylenbisphosphonat-Verbindung an Stelle des natürlichen Pyrophosphats herzustellen. Das Methylenbisphosphonat zeigt erhöhte Stabilität genen Phosphatasen und wurde als Inhibitor für ADP-Ribosylcyclase verwendet. Eine Stabilitätserhöhung gegenüber Hydrolyse war nicht das Ziel (J.T. Slama, G.M. Blackburn).

Die zugrunde liegende Aufgabe der vorliegenden Erfindung ist es daher, stabile bioanalytische Messsysteme, insbesondere zur Bestimmung von Glucose, bereitzustellen, die die Hydrolyseempfindlichkeit von NAD/NADP vermeiden und gleichzeitig als Coenzyme in Enzymreaktionen aktiv sind.

Diese Aufgabe wird gelöst durch ein Testelement zur Bestimmung eines Analyten, umfassend (i) ein Coenzym abhängiges Enzym, und (ii) als Coenzym eine Verbindung mit folgender allgemeiner Formel (I): 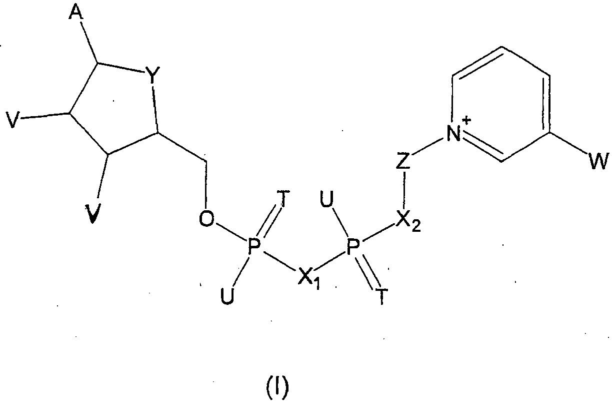 mit
- A =: Adenin oder ein Analog davon,
- T =: jeweils unabhängig O, S,
- U =: jeweils unabhängig OH, SH, BH₃⁻, BCNH₂⁻ ,
- V =: jeweils unabhängig OH oder eine Phosphatgruppe, wie in Anspruch 1 definiert,
- W =: COOR, CON(R)₂, COR, CSN(R)₂ mit R = jeweils unabhängig H oder C₁-C₂-Alkyl,
- X1, X2 =: jeweils unabhängig O, CH₂, CHCH₃, C(CH₃)₂, NH, NCH₃,
- Y =: NH, S, O, CH₂,
- Z =: ein Rest, umfassend eine cyclische Gruppe mit 5 C-Atomen, die gegebenenfalls ein Heteroatom ausgewählt aus O, S und N sowie gegebenenfalls einen oder mehrere Substituenten enthält, und einen Rest CR4₂, wobei CR4₂ an die cyclische Gruppe und an X2 gebunden ist, mit
- R4 =: jeweils unabhängig H, F, Cl, CH₃,
mit der Maßgabe, dass Z und der Pyridin-Rest nicht durch eine glycosidische Bindung verknüpft sind, wobei das Enzym eine wie in Anspruch 1 definierte Dehydrogenase ist,
oder ein Salz oder gegebenenfalls eine reduzierte Form davon.

In einer bevorzugten Ausführungsform ist W = CONH₂ oder COCH₃.

Bevorzugte Substituenten an Z sind ausgewählt aus der Gruppe OH, F, Cl sowie C₁-C₂-Alkyl, gegebenenfalls fluoriert oder chloriert oder/und OH-substituiert, O-C₁-C₂-Alkyl.

In einer bevorzugten Ausführungsform ist ein erster Rest V OH und ein zweiter Rest V eine Phosphatgruppe. Gegebenenfalls können die eine OH-Gruppe und die eine Phosphatgruppe mit den Kohlenstoffatomen, an welche sie gebunden sind, einen Zyklus bilden.

Überraschenderweise sind die erfindungsgemäßen Verbindungen Hydrolyse-stabil und gute Substrate in enzymatischen Nachweisverfahren und können zur biochemischen Diagnostik eingesetzt werden. Dieser Befund steht im Gegensatz zu dem der meisten der bisher bekannten NAD/NADP-Derivate, da diese Derivate in enzymatischen Nachweisverfahren in der Regel nur sehr kurz stabil sind.

Die Vorteile der erfindungsgemässen Verbindungen gegenüber dem Stand der Technik sind:
- hohe Stabilität,
- hohe enzymatische Aktivität,
- einfache und kostengünstige Synthese,
- Anwendbarkeit in allen bisher bekannten biochemischen Nachweisverfahren.

Durch die Bereitstellung stabiler NAD/NADP-Derivate mit der vorliegenden Erfindung bevorzugt in Kombination mit einer stabilisierenden Rezeptur, wie z.B. durch den Einschluss von Enzymen in Polymernetzwerke, können die Nachteile der bisher bekannten biochemischen Nachweisverfahren weitgehend vermieden werden. Weiterhin müssen keine stabilisierenden Zusätze eingesetzt werden. Dieses ist insbesondere vorteilhaft, da, je geringer die Anzahl der beteiligten, reaktiven Substanzen ist, die Chance höher ist, eine stabile Rezeptur für die Analyten-Bestimmung zu erhalten.

Mit der vorliegenden Erfindung werden Testelemente, umfassend eine Reihe stabiler NAD/NADP-Derivate, die für den Einsatz als Coenzym auf dem Testelement eine ausreichende enzymatische Aktivität besitzen, zur Verfügung gestellt.

Stabile NAD/NADP-Derivate können in allgemein bekannten Syntheseverfahren hergestellt werden. Hierzu wird von einem cyclischen Aminoalkohol die Aminogruppe via Zincke Chemie in das Pyridiniumderrivat überführt. Die primäre OH-Gruppe wird anschließend phosphoryliert und mit einem aktivierten AMP-Derivat zum NAD-Derivat gekoppelt. Alternativ kann auch die primäre OH-Gruppe zuerst phosphoryliert werden und dann die Aminogruppe mittels Zinckereaktion in einem Pyridin überführt werden.

Eine weitere Syntheseroute ist die Aktivierung des primären Alkohols zum Tosylat oder Jodid und die anschließende Alkylierung von ADP.

Bevorzugte Ausführungsformen des erfindungsgemäßen Testelements umfassen z.B. Verbindungen mit folgender allgemeiner Formel (I'): 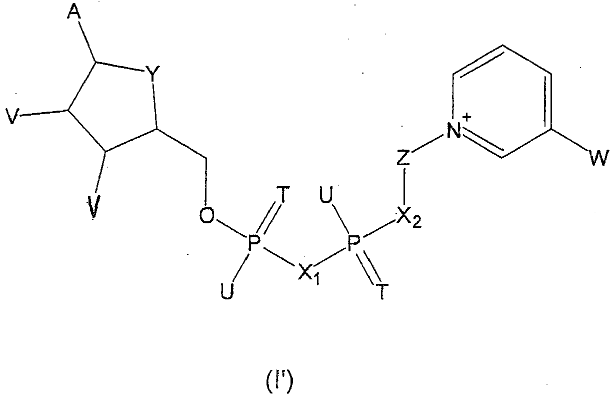 mit
- A =: Adenin oder ein Analog davon,
- T =: jeweils unabhängig O, S,
- U =: jeweils unabhängig OH, SH, BH₃⁻, BCNH₂⁻,
- V =: jeweils unabhängig OH oder eine Phosphatgruppe,
- W =: COOR, CON(R)₂, COR, CSN(R)₂ mit R = jeweils unabhängig H oder C₁-C₂-Alkyl,
- X1, X2 =: jeweils unabhängig O, CH₂, CHCH₃, C(CH₃)₂, NH, NCH₃,
- Y =: NH, S, O, CH₂,
- Z =: ein gesättiger oder ungesättigter carbocyclischer oder heterocyclischer Fünfring, insbesondere eine Verbindung der allgemeinen Formel (II) 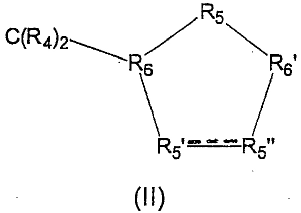
wobei zwischen R5' und R5" eine Einfach- oder Doppelbindung vorliegen kann,
- R4 =: jeweils unabhängig H, F, Cl, CH₃,
- R5 =: CR4₂,
wenn zwischen R5' und R5" eine Einfachbindung vorliegt, ist
- R5' =: O, S, NH, NC₁-C₂-Alkyl, CR4₂, CHOH, CHOCH₃,
- R5" =: CR4₂, CHOH, CHOCH₃.
wenn zwischen R5' und R5" eine Doppelbindung vorliegt, ist R5'=R5"=CR4, R6, R6' = jeweils unabhängig CH, CCH₃,
oder ein Salz oder gegebenenfalls eine reduzierte Form davon.

Ein weiterer hier beschriebener Gegenstand sind Verbindungen mit folgender allgemeiner Formel (I"): 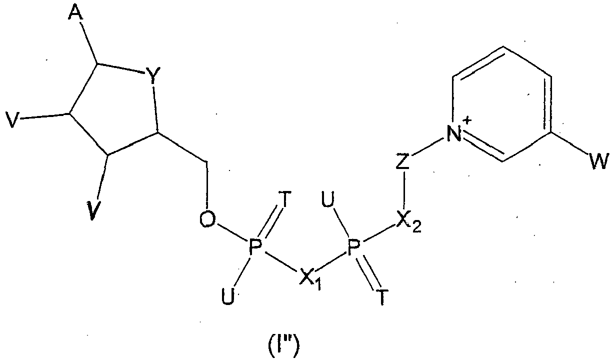 mit
- A =: Adenin oder ein Analog davon,
- T =: jeweils unabhängig O, S,
- U =: jeweils unabhängig OH, SH, BH₃⁻, BCNH₂⁻,
- V =: jeweils unabhängig OH oder eine Phosphatgruppe,
- W =: COOR, CON(R)₂, COR, CSN(R)₂ mit R = jeweils unabhängig H oder C₁-C₂-Alkyl,
- X1, X2 =: jeweils unabhängig O, CH₂, CHCH₃, C(CH₃)₂, NH, NCH₃,
- Y =: NH, S, O, CH₂,
- Z =: ein gesättiger oder ungesättigter carbocyclischer oder heterocyclischer Fünfring, insbesondere Verbindungen der allgemeinen Formel (II) 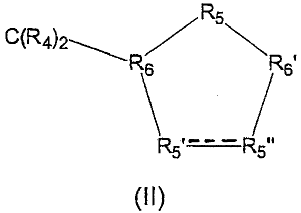
wobei zwischen R5' und R5" eine Einfach- oder Doppelbindung vorliegen kann,
- R4 =: jeweils unabhängig H, F, Cl, CH₃,
- R5 =: CR4₂,
wenn zwischen R5' und R5" eine Einfachbindung vorliegt, ist
- R5' =: O, S, NH, NC₁-C₂-Alkyl, CR4₂, CHOH, CHOCH₃,
- R5" =: CR4₂, CHOH, CHOCH₃,
wenn zwischen R5' und R5" eine Doppelbindung vorliegt, ist R5'=R5"=CR4, R6, R6' = jeweils unabhängig CH, CCH₃,
mit der Maßgabe, dass wenn R5 = CH₂, T = O, U = jeweils OH, V = OH, W = CONH₂, X = O und Y = O sind, dann sind R5' und R5" nicht gleichzeitig CHOH,
oder ein Salz oder gegebenenfalls eine reduzierte Form davon.

In einer bevorzugten Ausführungsform enthalten die Verbindungen Adenin-Analoga, wie z.B. C₈- und N₆-substituiertes Adenin, Deaza-Varianten wie 7-Deaza, Azavarianten wie 8-Aza oder Kombinationen wie 7-Deaza oder 8-Aza oder carbocyclische Analoga, wie Formycin, wobei die 7-Deazavarianten in der 7-Position mit Halogen, C₁-C₆-Alkinyl, -Alkenyl oder -Alkyl substituiert sein können.

In einer weiteren bevorzugten Ausführungsform enthalten die Verbindungen Adenosin-Analoga, welche statt Ribose z.B. 2-Methoxy-deoxyribose, 2'-Fluoro-deoxyribose, Hexitol, Altritol bzw. polycyclische Analoga, wie Bicyclo-, LNA- und Tricyclo-Zucker enthalten.

Insbesondere können auch (Di-)-Phosphatsauerstoffe isotronisch ersetzt sein, wie z.B. O- durch S- bzw. BH₃-, O durch NH, NCH₃ bzw. CH₂ und =O durch =S.

In einer bevorzugten Ausführungsform ist mindestens ein Rest U der Verbindung verschieden von OH und besonders bevorzugt ist mindestens ein Rest U = BH₃-,

Insbesondere bevorzugte Ausführungsformen sind Testelemente umfassend die Derivate Borano carba-NAD, c-Pentyl-NAD, Pyrrolyl-NAD, Furanyl-NAD, carba-NADcyclophosphat, carba-NADP, Pyrrolidinyl-NAD

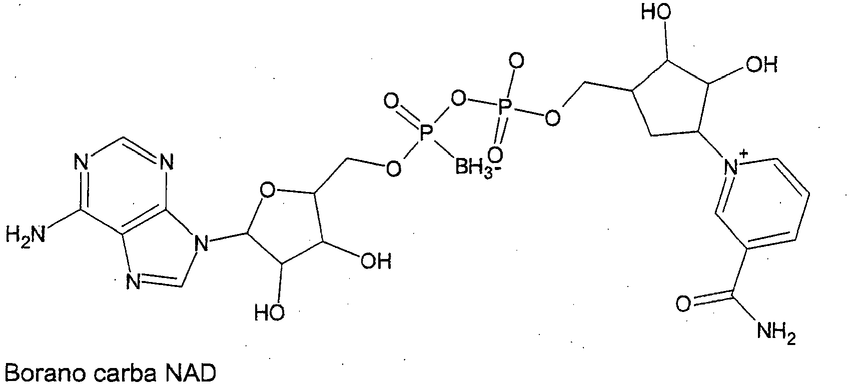

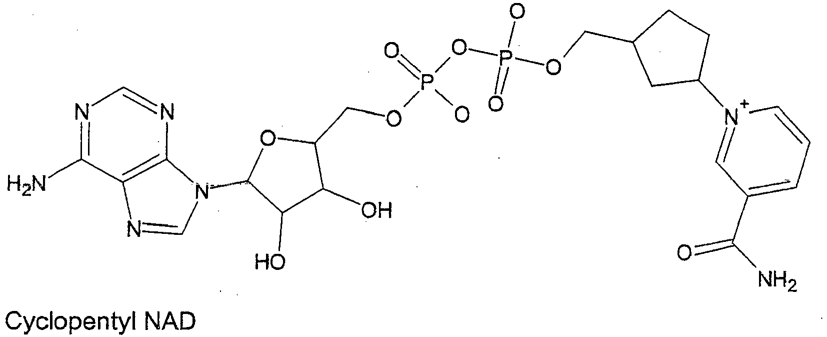

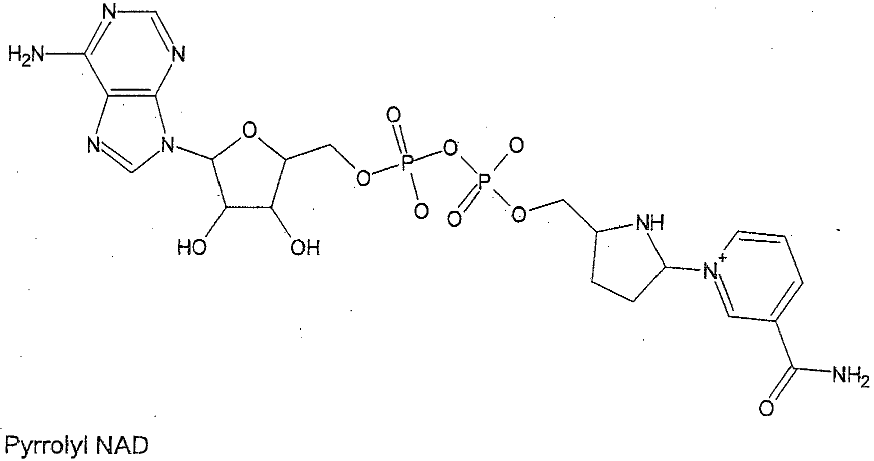

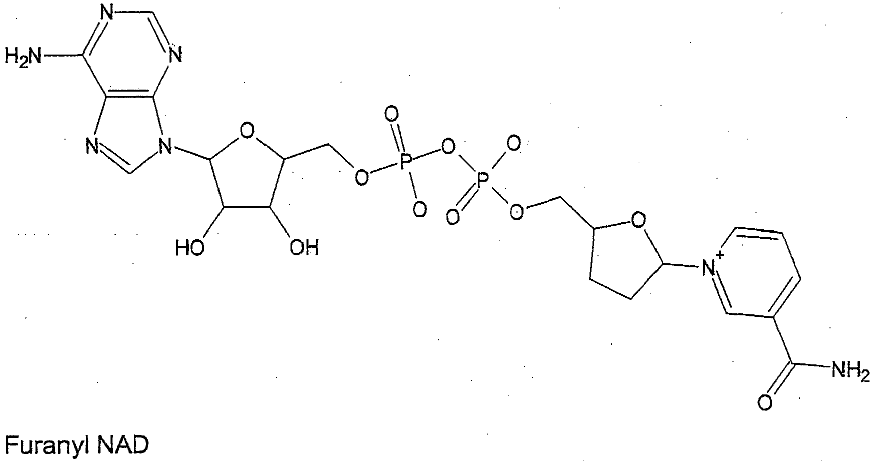

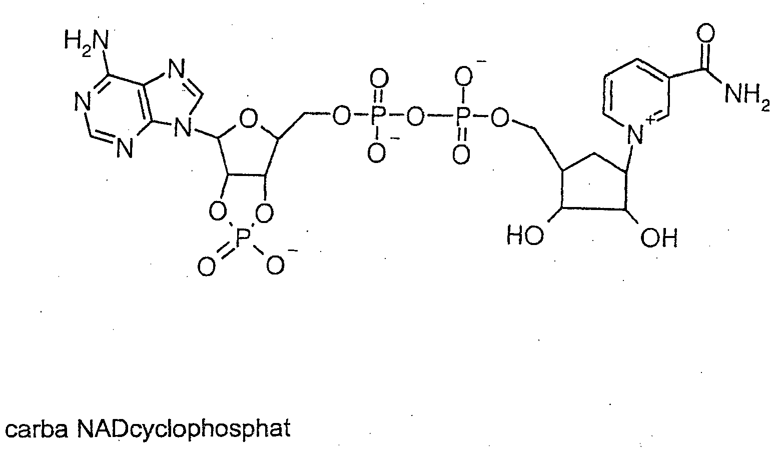

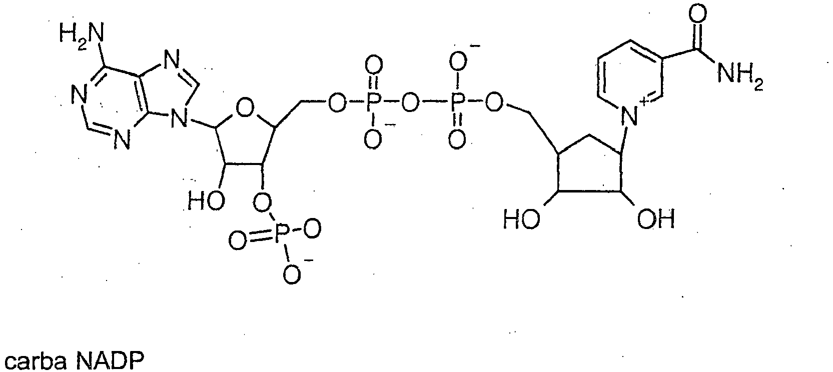

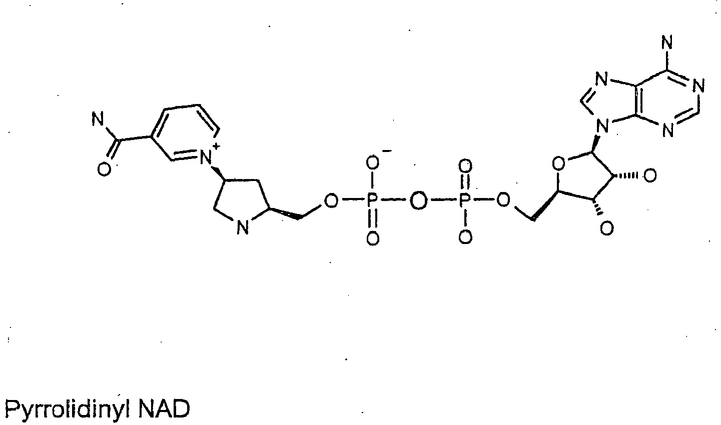

Biochemische Nachweise von Analyten, beispielsweise Parametern in Körperfiüssigkeiten, wie etwa Blut, Serum, Plasma oder Urin, bzw. in Abwasserproben oder Lebensmitteln spielen eine große Rolle in der Diagnostik. Dabei wird der zu bestimmende Analyt mit einem geeigneten Enzym und einem Coenzym in Kontakt gebracht.

Als Analyten können beliebige biologische oder chemische Substanzen bestimmt werden, die durch eine Redoxreaktion nachgewiesen werden können, z.B. Substanzen, bei denen es sich um Substrate eines Coenzymabhängigen Enzyms handelt oder Coenzym-abhängige Enzyme selbst. Bevorzugte Beispiele für Analyten sind Glucose, Milchsäure, Äpfelsäure, Glycerin, Alkohol, Cholesterin, Triglyceride, Ascorbinsäure, Cystein, Glutathion, Peptide, Harnstoff, Ammonium, Salicylat, Pyruvat, 5'-Nukleotidase, Creatinkinase (CK), Lactat-Dehydrogenase (LDH), Kohlendioxid etc.

Beim Nachweis von Enzymsubstraten enthält das Testelement eine Dehydrogenase, ausgewählt aus einer Lactat-Dehydrogenase (E.C.1.1.1.27, 1.1.1.28), Malat-Dehydrogenase (E.C.1.1.1.37), Glycerin-Dehydrogenase (E.C.1.1.1.6), alpha-Hydroxybutyrat-Dehydrogenase, Sorbitol-Dehydrogenase oder Aminosäure-Dehydrogenase, z. B. L-Aminosäure-Dehydrogenase (E.C.1.4.1.5).

Beim Nachweis von Enzymen enthält das Testelement vorzugsweise neben dem Coenzym ein zum Nachweis des Enzyms geeignetes Enzym-Substrat.

Die Veränderung des Coenzyms, d.h. der Verbindung, durch Reaktion mit dem Analyten (wenn der Analyt ein Enzymsubstrat ist) bzw. durch eine Analyt-katalysierte Reaktion (wenn der Analyt ein Enzym ist) kann grundsätzlich auf beliebige Art und Weise nachgewiesen werden. Hier können grundsätzlich alle aus dem Stand der Technik bekannten Methoden zum Nachweis enzymatischer Reaktionen eingesetzt werden. Vorzugsweise wird jedoch die Veränderung des Coenzyms durch optische Methoden nachgewiesen. Optische Nachweismethoden umfassen beispielsweise die Messung von Absorption, Fluoreszenz, Circulardichroismus (CD), optische Rotationsdispersion (ORD), Refraktometrie etc. Besonders bevorzugt wird die Veränderung des Coenzyms durch Messung der Fluoreszenz nachgewiesen. Die Fluoreszenzmessung ist hochsensitiv und ermöglicht den Nachweis selbst geringer Konzentrationen des Analyten in miniaturisierten Systemen.

Zum Nachweis eines Analyten kann ein Flüssigtest verwendet werden, wobei das Reagenz z.B. in Form einer Lösung oder Suspension in einer wässrigen oder nichtwässrigen Flüssigkeit oder als Pulver oder Lyophilisat vorliegt. Es kann jedoch auch ein Trockentest verwendet werden, wobei das Reagenz auf einem Träger, einem Teststreifen, aufgebracht ist. Der Träger kann beispielsweise einen Teststreifen, umfassend ein saugfähiges oder/und quellbares Material, umfassen, das von der zu untersuchenden Probeflüssigkeit benetzt wird.

Als Nachweisreagenz kann aber auch eine Gelmatrix mit einem darin eingelagerten Enzym-Coenzym-Komplex verwendet werden (siehe DE 102 218 45 A1).

Das Enzym kann hierbei entweder zusammen mit der erfindungsgemäßen Verbindung in die Matrix einpolymerisiert werden oder die Matrix kann nach der Polymerisation in Gegenwart des Enzyms mit einer Lösung des Coenzyms in Kontakt gebracht werden, so dass sich der entsprechende Enzym-Coenzym-Komplex bildet.

Ein weiterer hier beschriebener Gegenstand umfasst einen Reagenzienkit sowie dessen Verwendung zum Nachweis von Analyten. Der Reagenzienkit kann eine erfindungsgemäße Verbindung, ein geeignetes Enzym sowie einen geigneten Reaktionspuffer enthalten. Geeignete Enzyme wurden bereits beschrieben. Der erfindungsgemäße Reagenzienkit ist vielseitig anwendbar und kann zur Bestimmung von Analyten, wie z. B. Glucose, Milchsäure, Äpfelsäure, Glycerin, Alkohol, Cholesterin, Triglyceride, Ascorbinsäure, Cystein, Glutathion, Peptide, Harnstoff, Ammonium, Salicylat, Pyruvat, 5'-Nukleotidase, CK, LDH, Kohlendioxid etc., verwendet werden. Bevorzugt ist ein Reagenzienkit, welcher eine erfindungsgemäße Verbindung und Glucose-Dehydrogenase (E.C.1.1.1.47) enthält, zum Nachweis von Glucose im Blut.

Der Reagenzienkit kann zum Nachweis eines Analyten in einem Trocken- oder Flüssigtest verwendet werden.

Ein weiterer hier beschriebener Gegenstand umfasst einen Teststreifen zum fluorometrischen oder photometrischen Nachweis eines Analyten. Ein solcher Teststreifen enthält eine Verbindung wie zuvor angegeben als Coenzym und ein geeignetes Enzym oder ein Enzymsubstrat immobilisiert auf einem saugfähigen oder/und quellbaren Material. Geeignete Materialien können z.B. aus Cellulosen, Kunststoffen etc. ausgewählt werden.

Ein weiterer Gegenstand der vorliegenden Erfindung umfasst ein Verfahren zum Nachweis eines Analyten, umfassend die Schritte:
(a) Inkontaktbringen einer Probe mit einem erfindungsgemäßen Testelement, umfassend ein Coenzym und
(b) Nachweisen des Analyten, z.B. anhand der Veränderung des Coenzyms.

Ein weiterer Vorteil der Erfindung besteht darin, dass die Fluoreszenzemission der Coenzyme eine bathochrome Verschiebung zeigt, so dass eine geringe Interferenz mit der Fluoreszenzemission anderer Materialien des Testelements oder/und der Probe besteht.

Alle dargestellten bevorzugten Ausführungsformen eines Gegenstands der vorliegenden Erfindung sollen für die weiteren Gegenstände der Erfindung gelten, wie z.B. bevorzugte Ausführungsformen der erfindungsgemäßen Verbindungen.

Die Erfindung soll durch die nachfolgenden Figuren und Beispiele näher erläutert werden.

### Figuren

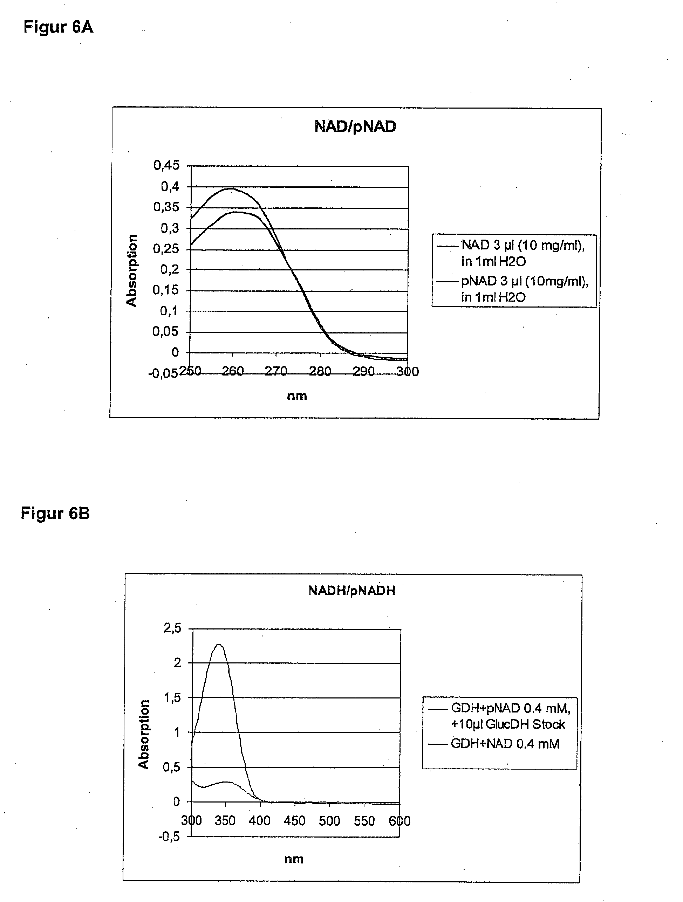

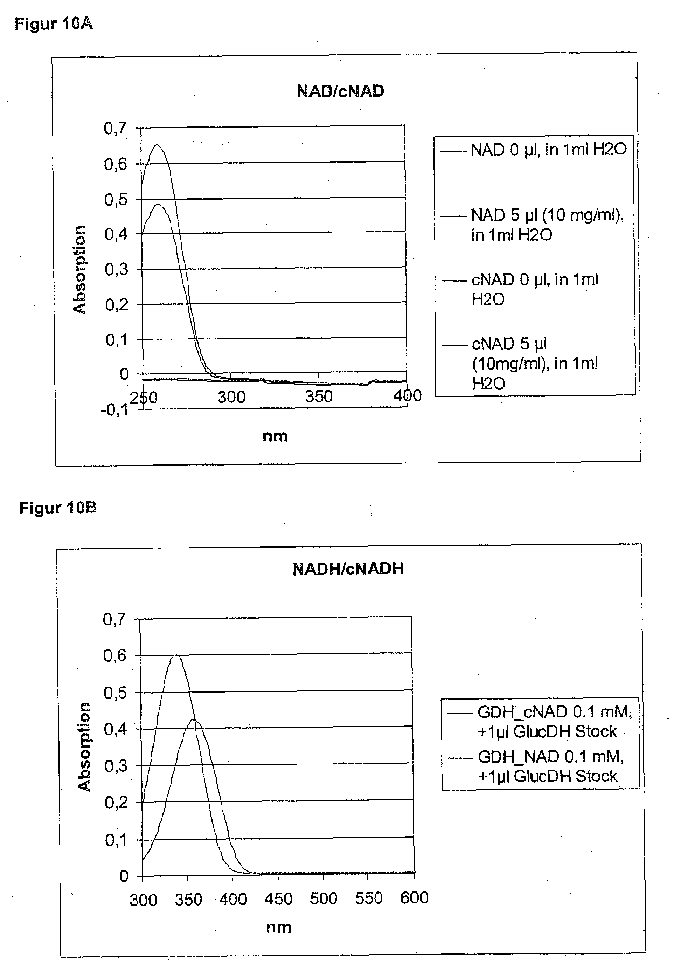

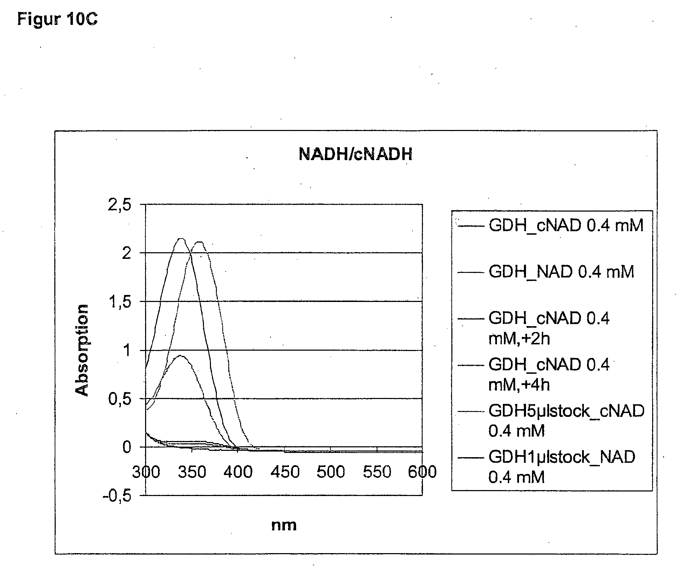

**Figur 1**
   Darstellung des Syntheseverfahrens von carbaNAD (cNAD).
**Figur 2**
   Darstellung der Ergebnisse der Belastung von NAD bei 8 °C und 37 °C
**Figur 3**
   Darstellung der Ergebnisse der Belastung von carbaNAD bei 8 °C und 37 °C
**Figur 4**
   Darstellung des Syntheseverfahrens von Borano NAD durch Alkylierung von ADP, mit Y = BH₃ nur Alkylierung an beta-Phosphat des ADP.
**Figur 5**
   Darstellung des Syntheseverfahrens von Pyrrolidinyl-NAD (pNAD). Neben den Strukturformeln sind Verbindungsnummern und Ausbeuten der jeweiligen Reaktionsschritte angegeben.
**Figur 6A/6B**
   Absorptionsspektren von NAD und pNAD (Figur 6A) und NADH bzw. pNADH (Figur 6B).
**Figur 7****:**
   Fluoreszenzpektren von NADH und pNADH als GlucDH-Komplex (Emissionsspektren)
**Figur 8**
   Fluoreszenzpektren von NADH und pNADH als GlucDH-Komplex (Anregungsspektren)
**Figur 9**
   Stabilitätsvergleich von NAD und pNAD
**Figur 10A/10B****/****10C**
   Absorptionsspektren von NAD und cNAD (Figur 10A) bzw. von NADH und cNADH (Figuren 10B und 10C)
**Figur 11**
   Fluoreszenzpektren von NADH und cNADH als GlucDH-Komplex

### Beispiele

### Experimentelle Herstellung stabiler NAD/NADH-Derivate

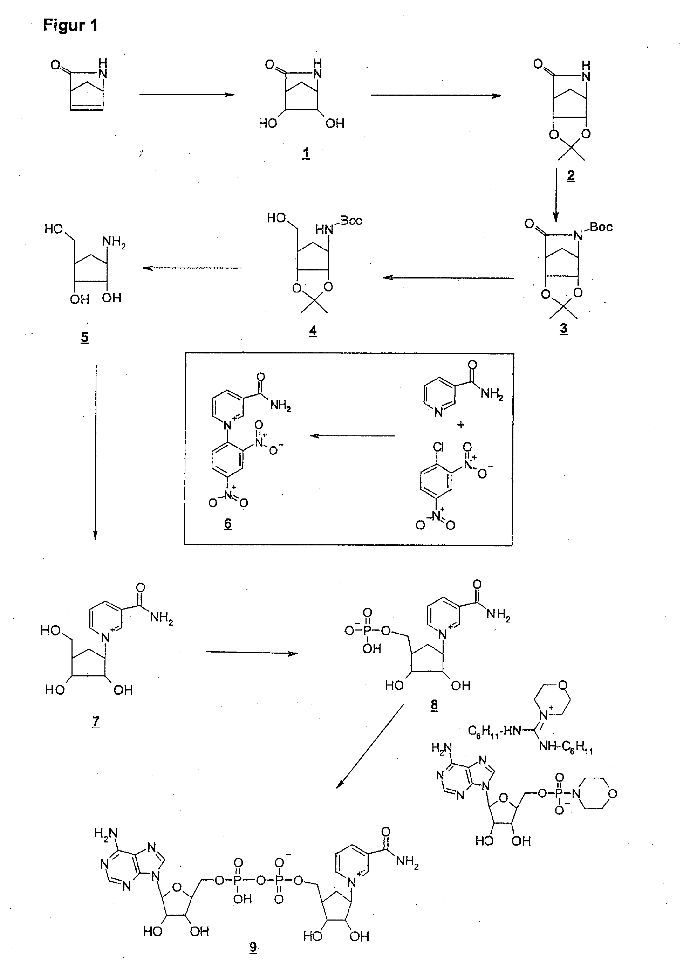

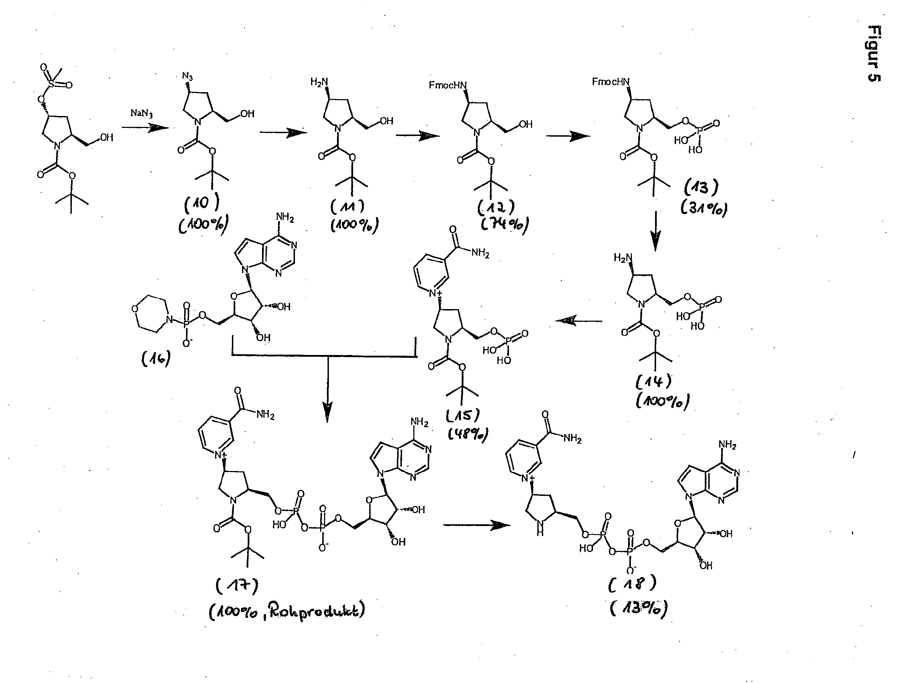

Die Herstellung stabiler NAD/NADH-Derivate wird exemplarisch an der Herstellung von carbaNAD (Verbindung 9, Figur 1) und Pyrrolidin-NAD (Verbindung 18, Figur 5) dargestellt. Weitere Derivate können mit entsprechenden Syntheseverfahren hergestellt werden. Die entsprechenden Aminoalkohole als Ausgangsreagenzien sind literaturbekannt:
2-Amino-1,4-Anhydro-2,3-Dideoxy-L-threo-Pentitol: Huryn, Donna M.; Sluboski, Barbara C.; Tam, Steve Y.; Todaro, Louis J.; Weigele, Manfred. Tetrahedron Letters (1989), 30(46), 6259-62.
3-Amino-, (1 R,3S)-Cyclopentanemethanol, Beres, J.; Sagi, G.; Tomoskozi, I.; Gruber, L.; Gulacsi, E.; Otvos, L.: Tetrahedron Letters (1988), 29(22), 2681-4

### A) Herstellung von carbaNAD

### I. 1R-(-)-exo-cis-5,6-Dihydroxy-2-azabicyclo[2.2.1]heptan-3-on (1).

In einem 1 I Rundkolben wird zu einer Lösung von 22,5 g (167 mmol) N-methylmorpholin-N-oxid in 80 ml deionisiertem Wasser eine Lösung von 16,4 g (147mol) 1R-(-)-2-Azabicyclo[2.2.1]hept-5-en-3-on in 400 ml Aceton gegeben. Unter Eiskühlung werden innerhalb von 15 min 15 ml (1,2 mmol) einer 2,5 % Lösung von Osmiumtetraoxid in tert-Butanol gegeben. Die Mischung wird anschließend bei Raumtemperatur über Nacht gerührt.

Die Lösemittel werden am Rotationsverdampfer abdestilliert. Es wird mit 100 ml gerührt und nochmals am Rotationsverdampfer abdestilliert. Anschließend wird in 600 ml deionisiertem Wasser gelöst und mit 35 g Aktivkohle versetzt. Die Mischung wird heftig für 1 h gerührt und dann über einen Seitz Tiefenfilter K 250 filtriert. Vom Filtrat wird das Wasser am Rotationsvedampfer abdestilliert. Das Produkt wird ohne weitere Reinigung eingesetzt.

DC (Merck Silica gel 60 F-254): Essigsäureethylester/Methanol/Eisessig 7:2:1 R_{f} 0,75 (Ausgangsmaterial), 0,53 (1). Anfärben mit TDM/Entwicklung Chlorkammer
* TDM Reagenz: Lösung 1: 10 g N,N,N',N'-Tetramethyl-4,4'-diaminodiphenylmethan in 40 ml Eisessig und 200 ml deionisiertem Wasser. Lösung 2: 20 g Kaliumchlorid in 400 ml deionisiertem Wasser. Lösung 3: 0,3 g Ninhydrin in 10 ml Eisessig lösen und 90 ml deionisiertes Wasser zugeben. Fertiges Reagenz: Mischung aus Lösung 1 und 2 und Zugabe von 6 ml von Lösung 3.

### II. 1R-(-)-exo-cis-5,6-Dimethylmethylendioxy-2-azabicyclo[2.2.1] heptan-3-on (2)

Das Rohprodukt 1 wird für 1 h in 200 ml absolutem Ethanol unter Rückfluss zum Sieden erhitzt Nach Zugabe von 400 ml (3,26 mol) Dimethoxypropan und 250 mg (2,2 mmol) Pyridinhydrochlorid wird die Mischung für weitere 15 min unter Rückfluss zum Sieden erhitzt. Nach Zugabe von 10 ml gesättigter Natriumhydrogencarbonatlösung wird die Lösung am Rotationsverdampfer unter Vakuum zur Trockene eingeengt. Der Rückstand wird mit 500 ml Chloroform, 150 ml gesättigter Kochsalzlösung und 75 ml gesättigter Natriumhydrogencarbonatlösung versetzt und in einen Scheidetrichter überführt. Nach Ausschütteln wird über Nacht stehengelassen, wobei die Phasentrennung stattfindet.

Die organische Phase wird abgetrennt und die wässrige Phase noch zweimal mit je 200 ml Chloroform extrahiert Die vereinigten organischen Phasen werden über Magnesiumsulfat getrocknet. Nach Abfiltrieren des Trockungsmittels wird das Lösungsmittel unter reduziertem Druck am Rotationsverdampfer abdestilliert. Das Rohprodukt (24,9 g = 92 %) wird ohne weitere Reinigung eingesetzt.

DC (Merck silica gel 60 F-254): Essigsäureethylester/Methanol/Eisessig 7:2:1 R_{f} 0,84. Anfärben mit TDM/Entwicklung Chlorkammer).

### III. 1R-(-)-4-N-tert-butyloxycarbonyl-exo-cis-5,6-dimethylmethylendioxy-2-azabicyclo[2.2.1]heptan-3-on (3)

Zu einer Lösung von 24,9 g (135,7 mmol) Rohprodukt 2 in 450 ml abs. Chloroform werden unter Argon 41,5 g (190 mmol) Di-tert-Butyldicarbonat und 0,83 g (6.8 mmol) 4-Dimethylaminopyridin gegeben. Die Mischung wird unter Rühren solange unter Rückfluss zum Sieden erhitzt, bis die Gasentwicklung aufhört. Die Mischung wird über eine Säule, die mit 40 g Silica Gel 60 gefüllt und mit Chloroform äquilibriert ist, gefiltert. Es wird mit 100 ml Chloroform gewaschen. Vom Filtrat wird das Lösungsmittel unter reduziertem Druck am Rotationsverdampfer abdestilliert. Das Rohprodukt wird 60 min bei 10 mbar und 40 °C getrocknet Es wird ohne weitere Reinigung eingesetzt.

DC (Merck Silica gel 60 F-254): Essigsäureethylester/Hexan 3:2 R_{f} =0,85. Anfärben mit TDM/Entwicklung Chlorkammer).

### IV. (-)-(1R,2R,3S,4R)-4-(N-tert-butyloxycarbonyl)amino-2,3-dimethylmethylendioxy-1-(hydroxymethyl)cyclopentan (4)

Bei Raumtemperatur wird das Rohprodukt 3 unter Rühren in 400 ml Tetrahydrofuran gelöst und 80 ml deionisiertes Wasser zugegeben. Nach Kühlen auf 4 °C werden auf einmal 5,3 g Natriumborhydrid zugegeben und über Nacht gerührt, wobei man die Mischung sich langsam auf Raumtemperatur erwärmen lässt. Es werden 100 ml Ethanol zugegeben und 6 h bei Raumtemperatur gerührt. Die Lösemittel werden unter reduziertem Druck am Rotationsverdampfer abdestilliert. Es werden 300 ml gesättigter Natriumchloridlösung und 650 ml Essigester zugeben und in einen Scheidetrichter überführt. Die organische Phase wird abgetrennt und die wässrige Phase nochmals mit 350 ml Essigester gewaschen. Die vereinigten organischen Phasen werden über Magnesiumsulfat getrocknet. Nach Abfiltrieren des Trockungsmittels wird das Lösungsmittel unter reduziertem Druck am Rotationsverdampfer abdestilliert. Das Rohprodukt (42,2 g) wird mittels Säulenchromatographie und Silica Gel 60 (Säule h = 93 cm, d = 10 cm) Eluent THF/Hexan 1:3, dann THF/Hexan 2:3) Fluss 3 l/h. Es werden 40 ml Fraktionen gesammelt. Die Fraktionen werden mit DC (Merck Silica Gel 60 F-254: Essigsäureethylester/Hexan 3:2 R_{f} = 0.45. Anfärben mit TDM/Entwicklung Chlorkammer) kontrolliert. Von den vereinigten Produktfraktionen wird das Lösungsmittel am Rotationsverdampfer im Vakuum abdestilliert, Ausbeute: 24,9 g.

### V. (-)-(1R,2R,3S,4R)-4-Amino-2,3-dihydroxy-1-(hydroxymethyl)cyclopentan (5)

Zu 11,09 (38,6 mmol) 4 werden 8 ml deionisiertes Wasser und dann 80 ml Trifluoroessigsäure gegeben. Es wird für 6 h bei Raumtemperatur kräftig gerührt, wobei sich eine klare, schwach gelbe Lösung bildet. Es werden 200 ml deionisiertes Wasser hinzugegeben und unter Vakuum am Rotationsverdampfer evaporiert. Es werden nochmals 200 ml deionisiertes Wasser hinzugegeben und wieder unter Vakuum am Rotationsverdampfer evaporiert Das Rohprodukt wird in 100 ml deionisiertem Wasser im Ultraschallbad gelöst und filtriert. Das Filtrat wird auf eine Dowex 1X8 (100-200 mesh, OH- Form) lonenaustauschersäule (15 x 4,9 cm) aufgetragen und mit Wasser eluiert, wobei das Produkt nach ca. 150 ml in einem Volumen von 300 ml eluiert. (pH 10,4). Die Fraktionen werden mit DC (Merck Silica Gel 60 F-254: Butanol/Eisessig/Wasser 5:2:3 R_{f} = 0,42, Anfärben mit TDM/Entwicklung Chlorkammer) kontrolliert. Von den vereinigten Produktfraktionen wird das Lösungsmittel am Rotationsverdampfer im Vakuum abdestilliert, Ausbeute: 5,2 g farbloses Öl.

### VI. Zincke Salz des Nicotinamids (6)

Unter Stickstoff werden 58,6 g Dinitrochlorbenzol geschmolzen und dann zu der Schmelze 29,32 g Nicotinamid gegeben. Es wird für 2,5 h bei 110 °C erhitzt. Durch einen Rückflusskühler werden 500 ml eines 3:2(v/v) Ethanol/Wasser-Gemisches zugegeben und unter Rückfluss zum Sieden erhitzt bis eine Lösung entsteht. Nach Rühren über Nacht bei Raumtemperatur werden 150 ml 50 % Ethanol/Wasser und 100 ml Wasser zugegeben, in eine Scheidetrichter überführt und dreimal mit je 500 ml Chloroform gewaschen. Die abgetrennte wässrige Phase wird mit 300 ml und 50 g Aktivkohle versetzt, für 1 h bei Raumtemperatur gerührt und dann über einen Seitz Tiefenfilter K 700 filtriert. Das Filtrat wird am Rotationsvedampfer im Vakuum auf ca. 100 ml konzentriert, wobei die Badtemperatur nicht über 20 °C steigen darf. Es wird mit 300 ml Wasser verdünnt und unter Rühren bei Raumtemperatur werden 70 g Natriumtetrafluorborat zugegeben. Das Präzipitat wird aus Methanol/Wasser umkristallisiert. Das Kristallisat wird abfiltriert mit wenig Aceton und dann mit Diethylether gewaschen und im Hochvakuum bei 40 °C 24 h getrocknet, Ausbeute 21,1 g 23 %). Die Fraktionen werden mit DC (Merck Silica Gel 60 F-254: Butanol/Eisessig/Wasser 5:2:3 R_{f} = 0,56) kontrolliert.

### VII. (-)-(1R,2R,3S,4R)-4-(3-Carboxamidopyridin-1-yl)-2,3-dihydroxy-1-(hydroxymethyl)cyclopentan (6) = Carba Nicotinamidmononukleosid = carbaNMN

Unter Rühren bei Raumtemperatur wird eine Lösung von 4,5 g (31 mmol) Cylcopentylamin 5 in 110 ml abs. Methanol innerhalb 90 min zu einer Lösung von 15,3 g (40,7 mmol) des Zincke Salzes 6 in 110 ml abs. Methanol getropft. Es wird 1 ml Diisopropylethylamin zugegeben und dann zwei Tage bei Raumtemperatur gerührt. Es werden 500 ml Wasser zugegeben, in einen Scheidetrichter überführt und zweimal mit je 200 ml Methylenchlorid gewaschen. Von der abgetrennte wässrigen Phase wird das Wasser am Rotationsverdampfer unter Vakuum abdestilliert. Der Rückstand wird in 100 ml Wasser aufgenommen und mittels Säulenchromatographie Sephadex C25 (Na+ form) gereinigt: Säule 70 x 7,5 cm Elution von Puffer A (deionisiertes Wasser) auf Puffer B) 0,35 M NaCl in Wasser, Fluss 200 ml/h. Es werden 15 ml Fraktionen gesammelt und mit DC kontrolliert (Merck silica gel 60 F-254: Butanol/Eisessig/Wasser 5:2:3 Rf 0,22).

Von den vereinigten Produktfraktionen wird das Lösungsmittel am Rotationsverdampfer im Vakuum abdestilliert. Der salzhaltige Rückstand wird mit 500 ml heißem Ethanol ausgekocht. Es wird heiß filtriert und 12 h bei Raumtempertur stehen gelassen. Der Niederschlag wird abfiltriert und vom Filtrat das Lösungsmittel unter Vakuum am Rotationsverdampfer abdestilliert. Ausbeute 7 g.

### VIII. (-)-(1R,2R,3S,4R)-4-(3-Carboxamidopyridin-1-yl)-2,3-dihydroxy-1-phosphatoylmethyl)cyclopentan (6) = carba NMN-Monophosphat

Zu einer Suspension von 7 g (27,7 mmol) carba NMN in 80 ml trockenem Phosphorsäuretrimethylester wird bei 0 °C eine Mischung aus 20 ml Phosphoroxychlorid und 50 ml Phosphorsäuretrimethylester gegeben. Es wird 2 h bei 0 °C und dann 2 h bei Raumtemperatur gerührt. Unter Eiskühlung werden 300 ml Wasser zugegeben und die Mischung am Rotationsverdampfer unter Vakuum auf 10 ml eingeengt. Es wird in 100 ml Wasser aufgenommen, filtriert und mittels Säulenchromatographie an Sephadex C25 (NEt3H+ Form) gereinigt: Säule 66 x 9 cm Elution von Puffer A (deionisiertes Wasser) auf Puffer B) 0,60 M Ammoniumacetat, Fluss 200 ml/h. Es werden 15 ml Fraktionen gesammelt und mit DC kontrolliert (Merck silica gel 60 F-254 plates: Isobuttersäure/Ammoniak/Wasser 66:1:33, Rf 0,25). Von den vereinigten Produktfraktionen wird das Lösungsmittel am Rotationsverdampfer im Vakuum abdestilliert. Der Rückstand wird in 100 ml Wasser gelöst und lyophilisiert. Diese Prozedur wird dreimal wiederholt. Ausbeute: 4,0 g.

### IX. carbaNAD (9)

Bei Raumtemperatur wird innerhalb 1 h eine Lösung von 1,25 g (30 mmol) AMP Morpholidat in 40 ml absolutem DMF zu einer Mischung aus einer Lösung von 3,31 g (10 mmol) carbaNMN-Monophosphat in 40 ml absolutem DMF und 78 ml (39 mmol) 3,5 % Tetrazol in abs. Acetonitril getropft. Die Mischung wird 2 Tage bei Raumtemperatur gerührt.

Unter Kühlung mit Trockeneis/Aceton wird der pH mit einer wässrigen 10 % KHCO₃-Lösung auf 6,5 eingestellt. Es wird mit 500 ml Wasser verdünnt und im Vakuum am Rotationsverdampfer vorsichtig bis zur Trocknung eingeengt. Der Rückstand wird in 150 ml deionisiertem Wasser gelöst und mittels Säulenchromatographie an Sephadex QAE 25 (NEt3H+ Form) gereinigt: Säule 65 x 4,5 cm. Elution von Puffer A (deionisiertes Wasser) auf Puffer B) 1M Triethylammoniumcarbonat, Fluss 200 ml/h: Es werden 15 ml Fraktionen gesammelt und mit DC kontrolliert (Merck silica gel 60 F-254 plates: Isobuttersäure/Ammoniak/Wasser 66:1:33, Rf 0,47).

Von den vereinigten Produktfraktionen wird das Lösungsmittel am Rotationsverdampfer im Vakuum abdestilliert Der Rückstand wird in 100 ml Wasser gelöst und lyophilisiert. Diese Prozedur wird dreimal wiederholt. Ausbeute: 1,1 g.

### Stabilitätsuntersuchung von carbaNAD

Eine 10 mM Lösung von carbaNAD bzw NAD wird bei pH 8 in 0,1 M Kaliumphosphatpuffer belastet. Der Gehalt wird nach 0,25, 75 und 175 h mittels HPLC-Chromatographie ermittelt.
Puffer A: 100 mM KHPO₄ + 10 mM Tetrabutylammoniomhydrogensulfat, pH 6,9
Puffer B: Puffer A + Acetonitril 1:1
Flussrate 1,0 ml/min Detektion: 254 nm
RP18 Säule L 125 diameter 4,6 mm
Gradient: in 40 min auf 35 % Puffer B halten, für 2 min dann innerhalb 3 min auf 0 % Puffer A

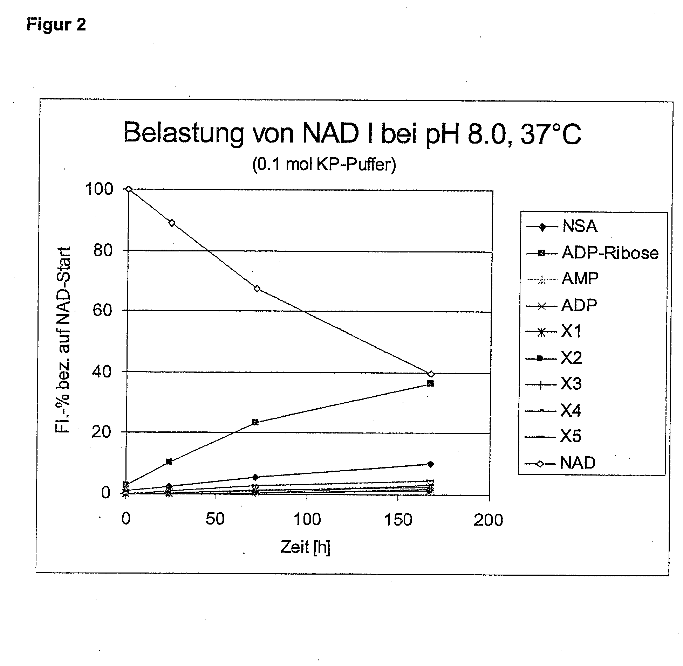

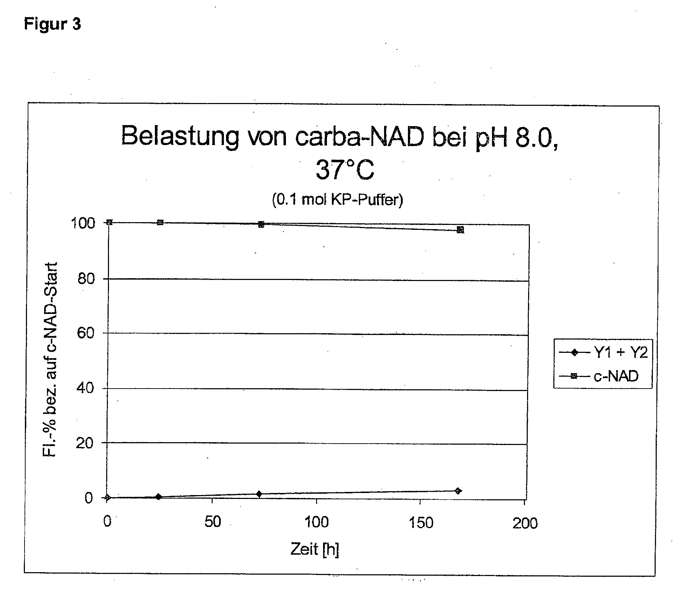

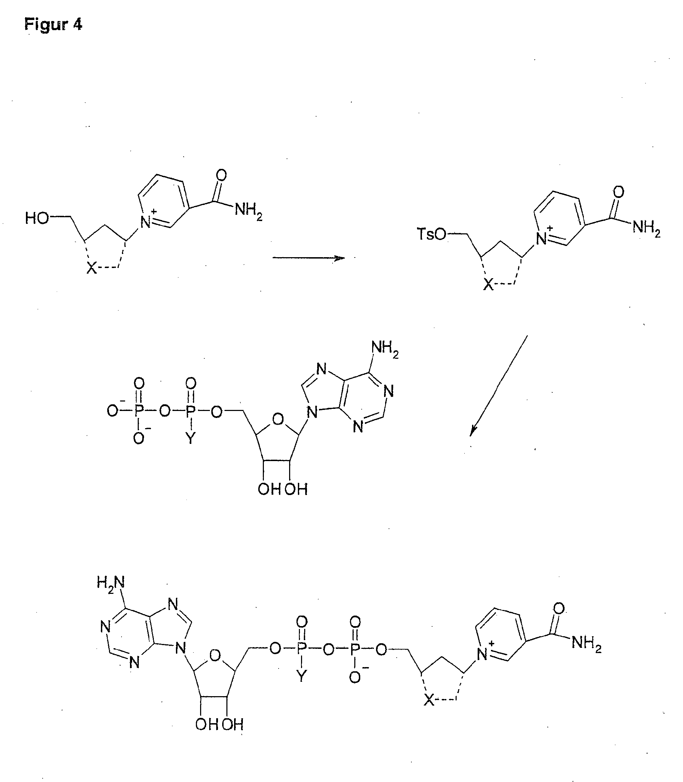

In den Figuren 2 und 3 werden die HPLC Flächenprozente nach Belastung zu unterschiedlichen Zeiten dargestellt.

Anhand des Auftretens von Zersetzungsprodukten (Nicotinsäureamid, ADP-Ribose, AMP, ADP und die unbekannten Zersetzungsprodukte für NAD sowie die unbekannten Zersetzungsprodukte Y1 und Y2 für cNAD) zeigt sich, dass cNAD im Vergleich zu NAD sehr stabil ist.

### B) Herstellung von Pyrrolidinyl-NAD

### I. Synthese pNAD 1. Stufe (Verbindung 10)

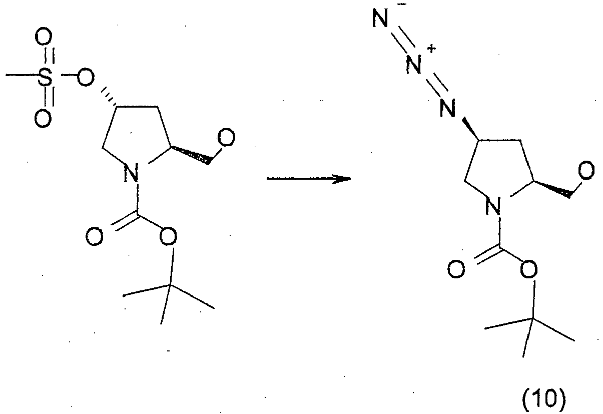

trans-N-t-BOC-O-mesyl-4-hydroxy-L-prolinol (35,4 g, 120 mmol) wurden in 500 ml DMF gelöst vorgelegt, Natriumazid (15,6 g, 240 mmol) gelöst in 75 ml Wasser zugegeben und 5 h auf 70 °C erwärmt. Anschließend wurde über Nacht bei Raumtemperatur nachgerührt, das Gemisch in 1000 ml ges. Kochsalzlösung gegossen und mit Essigester extrahiert. Der Essigester wurde mit Na₂SO₄ getrocknet und anschließend eingedampft.

Es ergaben sich 32,8 g (> 100 %) Rückstand (theoretischer Wert: 29 g).

Das Rohprodukt wurde nach DC und MS-Kontrolle direkt weiter verarbeitet. Zur Kontrolle wurde Dünnschichtchromatographie auf einer KG 60 F254 Platte (Laufmittel: Essigester/besprüht mit Ninhydrin) durchgeführt:
trans-N-t-BOC-O-mesyl-4-hydroxy-L-prolinol RF. 0,49
Produkt RF. 0,78
MS ESI ES+ 242
Die Identität des Produkts wurde ferner durch NMR-Untersuchung bestätigt.

### *trans-N-t-BOC-O-mesyl-4-hydroxy-L-prolinol ist käuflich erhältlich bei Sanochemia Pharmazeutika AG, Kat.-Nr. P -719

### II. Synthese pNAD 2. Stufe (Verbindung 11)

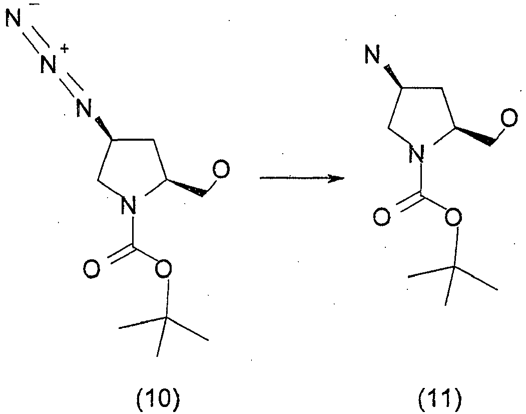

Verbindung 10 (120 mmol) wurde in 500 ml Methanol mit 2,0 g Pd-Kohle (10%ig) vermischt und 12 h bei 30 mbar hydriert. Dabei wurde der Reaktionskolben mehrmals mit H₂ gespült, der Katalysator abgesaugt und eingedampft.

Es ergab sich ein farbloses Öl (sollte wegen hoher Luftempfindlichkeit sofort weiter verarbeitet werden).
MS ESI ES+217 vorhanden
DC (Isohexan/Essigester 1/1 / KG 254 F/Ninhydrin): Produkt bleibt am Start
Die Identität des Produkts wurde ferner durch NMR-Untersuchung bestätigt.

### III. Synthese pNAD 3. Stufe (Verbindung 12)

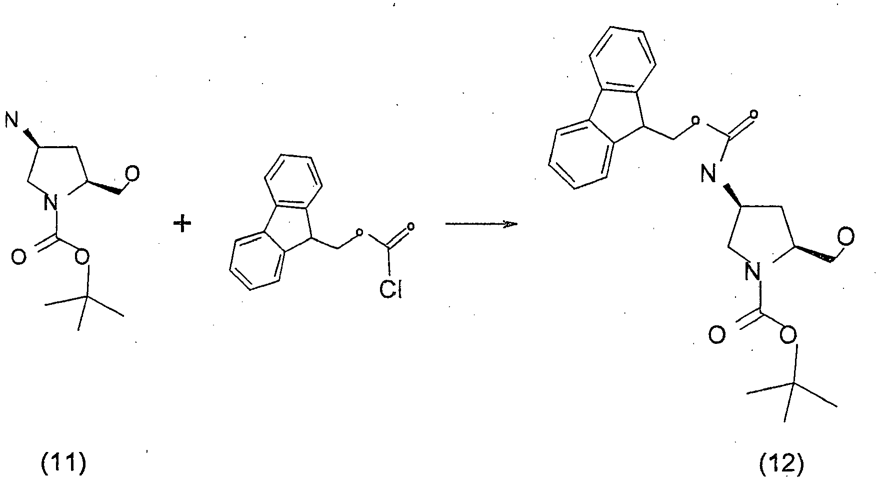

120 mmol der Verbindung 11 (MG: 216,28) wurden in 500 ml Dioxan mit NaHCO₃ (11,0 g, 130 mmol) und Fmoc-chlorid (33,8 g, 130 mmol) vermischt und über Nacht bei Raumtemperatur gerührt. Sich dabei ergebende Salze wurden abfiltriert, die Lösung eingedampft und der Rückstand über eine Kieselgel-Säule (Isohexan und Isohexan/EE 8/2 - 1/1) gereinigt.
Die Hauptfraktion ergab 39,0 g = 74,1 % * (theoretischer Wert = 52,6 g). DC (KG 60 F254 Laufmittel Isohexan/Essigester 2:1): RF 0,13
MS ESI ES + 439 / + 339
Die Identität des Produkts wurde ferner durch NMR-Untersuchung bestätigt.

### *Ausbeute bezieht sich auf Edukt 1. Stufe

### IV. Synthese pNAD 4. Stufe (Verbindung 13)

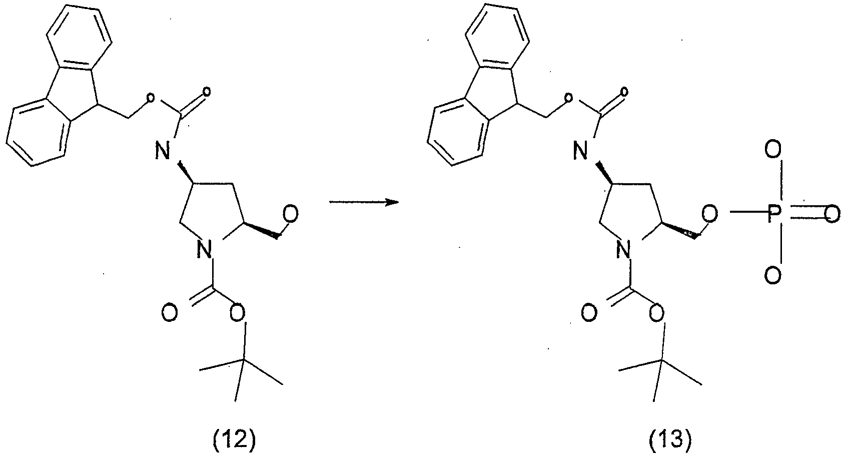

Verbindung 12 aus Stufe 3 (7,08 g, 16,1 mmol) wurde in 80 ml Trimethylphosphat gelöst und anschließend im Eisbad auf 0 °C gekühlt. POCl₃ mit Trimethylphosphat gemischt (13 ml frisch destilliertes POCl₃ in 13 ml Trimethylphosphat) wurde in einen Tropftrichter geben und unter Argon innerhalb von 20 min portionsweise dazugeben. Die Temperatur stieg in einer exothermen Reaktion bis auf 5 °C an. Anschließend wurden 2,6 ml Pyridin dazugeben und 40 min bei 0 °C und unter Argon nachgerührt.

Die Reaktionslösung wurde in 800 ml eisgekühlte 1 M Triethylammoniumhydrogencarbonat-Lösung (pH = 8) vorsichtig eingetropft. Nach vollständiger Zugabe wurde noch 1 h weiter gerührt. Die leicht trübe Lösung wurde anschließend auf 11 gesättigte NaCl-Lösung getropft (schnell). Zum besseren Auskristallisieren wurde über Nacht nachgerührt. Der Niederschlag wurde abfiltriert. Der Rückstand wurde über eine Diaion-Säule entsalzt. Dazu wurden 500 g Diaion in Isopropanol/Wasser 1/1 gegeben und man ließ über Nacht quellen. Diaion wurde in die Säule gefüllt und mit Wasser gespült. Der Rückstand wurde in 100 ml Wasser pH 3,5 (Essigsäure) angeschlämmt, anschließend auf eine Säule aufgetragen und so lange mit Wasser (pH 3,5) gespült bis er frei von Kochsalz war. Dann wurde die Substanz aus der Säule mit 25 % Isopropanol (pH 3,5) eluiert. Die Lösung wurde im Hochvakuum bei Raumtemperatur eingedampft.

Rückstand = 2,6 g = 31,3 %
DC RP8 F254 / MeOH / Wasser 9/1
MS ESI ES - 517,13
Die Identität des Produkts wurde ferner durch NMR-Untersuchung bestätigt.

### V. Synthese pNAD 5. Stufe (Verbindung 14)

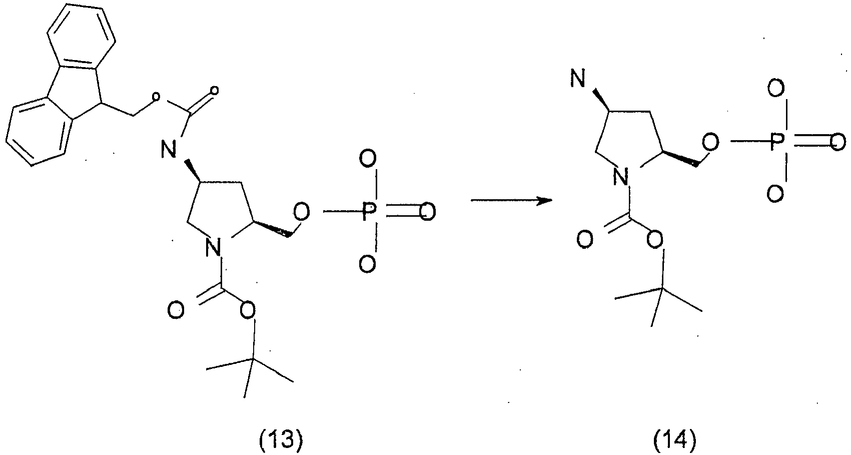

Ein Gemisch aus Verbindung 13 aus Stufe 4 (4,10 g, 7,9 mmol) in 250 ml Methanol und 83 ml 25 % Ammoniak wurde über Nacht bei Raumtemperatur gerührt und bei Raumtemperatur im Vakuum eingedampft. Der Rückstand wurde in 200 ml Wasser aufgenommen und 3 x mit 100 ml Essigester ausgerührt. Unlösliche Teile wurden abfiltriert, die klare Wasserphase abgetrennt und wiederum bei Raumtemperatur eingedampft.

Rückstand = 2,56 g = 100 %
MS ESI ES - 295

Zum Entfernen der NH₃-Kationen wurde der Rückstand 2 x in Hünigbase gelöst und im Hochvakuum jeweils wieder eingedampft

### VI. Synthese pFAD 6. Stufe (Verbindung 15)

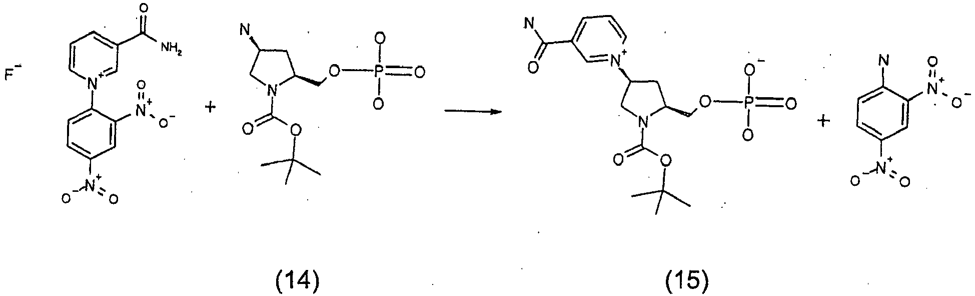

Zinke-Salz (2,66 g, 8,99 mmol) wurde angelöst in 50 ml Methanol vorgelegt und unter Rühren Verbindung 14 aus Stufe 5 (2,56 g, 8,31 mmol) gelöst in 50 ml Methanol zugetropft. Die Mischung färbte sich rot und ging allmählich in Lösung. Es wurde über Nacht bei Raumtemperatur nachgerührt und ausgefallener Niederschlag abfiltriert. Das Filtrat wurde im Vakuum eingedampft, in 100 ml Wasser aufgenommen und mit Essigester 3 x extrahiert.

Die Essigesterphase enthielt das Nebenprodukt Dinitroanilin, die Wasserphase enthielt Produkt und restliches Zincke Salz. Die Wasserphase wurde im Vakuum bei Raumtemperatur eingedampft und der erhaltene Rückstand mit 10 ml Wasser versetzt, 10 min auf einem Magnetrührer und gerührt und unlösliche Teile abfiltriert. Das Produkt blieb gelöst. Diese Lösung wurde auf eine mit Wasser gespülte Diaion HP20-Säule (1000 ml) gegeben und 2 x mit 1000 ml Wasser gespült. Anschließend wurde mit Wasser/5 % Isopropanol gespült und positive Fraktionen (detektiert mit DC RP8 MeOH/W 9/1) bei Raumtemperatur eingedampft. Der Rückstand wurde mit Isopropanol verrieben und mit Hilfe von Diethylether abgesaugt.

Rückstand = 1,60 g = 47,9 %
DC RP8 254 MeOH/W 9/1
MS ES - 400,1 / ES + 402,0 -zeigt auch doppelte Masse
Die Identität des Produkts wurde ferner durch NMR-Untersuchung bestätigt.

### VIIa. Synthese pNAD 7a. Stufe (Verbindung 16)

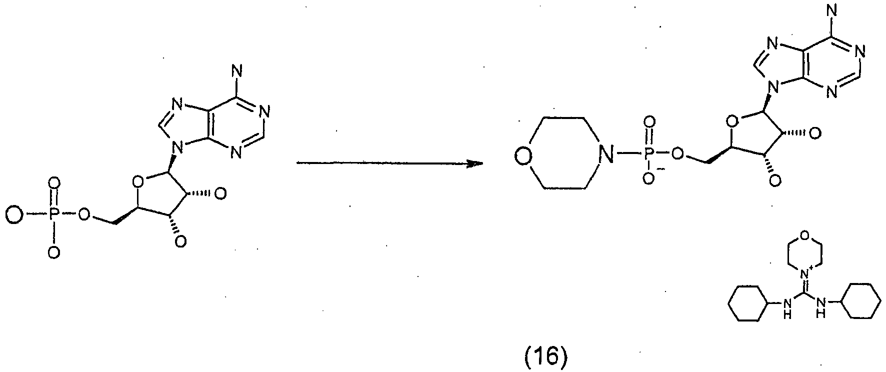

Ein Gemisch aus AMP-Säure (Adenosinmonophosphat-frei Säure) (10 g, 27,5 mmol) in 60 ml Methanol (getrocknet mit Natrium) und 5,3 ml (60 mmol) Morpholin (frisch destilliert) wurde solange gerührt bis eine klare Lösung entstand. Anschließend wurden 17 g (82,5 mmol) N,N'-Dicyclohexylcarbodiimid (DCC) zugegeben und über Nacht bei Raumtemperatur unter Feuchtigkeitsausschluss gerührt. Der entstandene Niederschlag (DCH) wurde abgesaugt und das Filtrat bei 30 °C einrotiert. Anschließend wurde mit 150 ml H₂O/150 ml Diethylether ausgerührt und nochmals filtriert. Nach Phasentrennung wurde die wässrige Phase noch zweimal mit je 75 ml Diethylether extrahiert. Die wässrige Phase wurde anschließend bei Raumtemperatur einrotiert. Den Rückstand wurde noch zweimal in Pyridin gelöst und jeweils wieder im Hochvakuum abrotiert.

### VII. Synthese pNAD 7. Stufe (Verbindung 17)

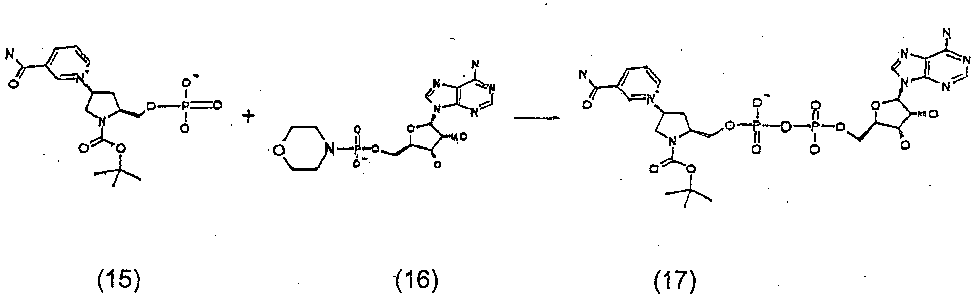

Ein Gemisch aus AMP-morpholidat (Verbindung 16 aus Stufe 7a) (2,53 g, 3,61 mol), Verbindung 15 aus Stufe 6 (1,60 g, 3,98 mmol), MnCl₂-Lösung in Formamid 0,2 M* (27,1 ml, 5,42 mmol) und MgSO₄ wasserfrei (0,87 g, 7,95 mmol) wurde über Nach bei Raumtemperatur gerührt und war nach dieser Zeit wie durch DC bestimmt (RP8 MeOH/W 9/1) großteils umgesetzt. Das Reaktionsgemisch wurde mit Acetonitril ausgefällt und abgesaugt.

Rückstand = 5,3 g (Th. 2,64 g)**
MS, ESI ES - 729,3 = Produkt, ES -415 = Kation von AMP-morpholidat, ES
-400,2/ES +402,1 Reste von Verbindung 15 (Stufe 6)
DC RP 8 F254 RF 0,085

*Zur Herstellung dieser Lösung wurden 2516 mg MnCl₂ wasserfrei unter Rühren gelöst in 100 ml Formamid 99,99%ig und anschließend mit 4A Molekularsieb versetzt.

**Der Rückstand wurde als Rohprodukt weiter verarbeitet.

### VIII. Synthese pFAD 8. Stufe (Verbindung 18, Pyrrolidinyl-NAD)

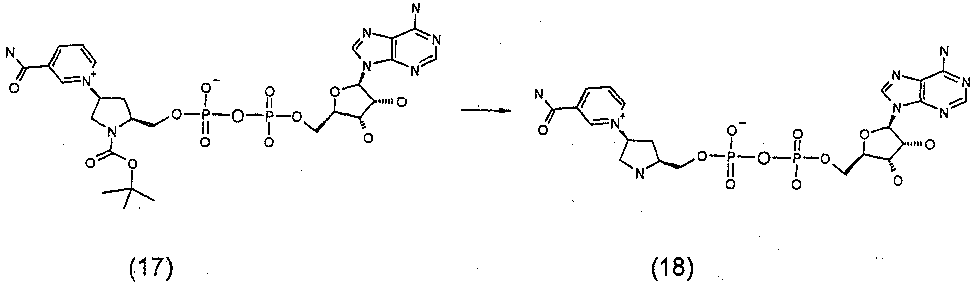

500 mg Verbindung 17 aus Stufe 7 (Rohprodukt, enthält ca. 50 % Salze) wurden mit 5,0 ml Trifluoressigsäure (TFA) versetzt und 1 h bei Raumtemperatur gerührt und anschließend eingedampft. Als Rückstand ergaben sich etwa 500 mg farbloses Öl.
MS ESI ES - 729,24 (Zusatz von NH₃ erforderlich)

Die Aufreinigung erfolgte in 2 Portionen von 200 mg und 300 mg in jeweils 2 Trennschritten:

### 1.Trennschritt:

Fraktogel EMD SO3- s Säule: D (innen) = 14 mm L (Packung) = 85 mm
   I. Konditionierung (Flussrate 5 ml/min)
      a) 100 ml H₂O
      b) 200 ml 0,25 M H₂SO₄
      c) 100 ml H₂O
      d) 200 ml 1 M Ammoniak-Lösung
      e) 100 ml H₂O
   II. Trennung :
      a) 200 mg Substaz gelöst in 5 ml H₂O aufgegeben
      b) eluiert mit Gradient H₂O → 0,2 M NH₄HC₃-Lsg. *(Laufmiftel A* = 250 ml H₂O in Erlenmeyerkolben vorgelegt und auf Magnetrührer gerührt, mit 5 ml/min auf Säule gepumpt *Laufmifel B* = 0,2 M NH₄HCO₃-Lsg. Mit 2,5 ml/min zu A gepumpt.)
   III. Fraktionierung:
      a) Fraktionen von jeweils 3 ml
      b) 1. Peak Verunreinigungen
      c) 2. Peak nach ca. 70 ml Vorlauf Substanz
   IV. Rekonditionierung:
      a) 100 ml 1 M Ammoniak-Lsg.
      b) 100 ml H₂O

### 2. Trennschritt:

Diaion HP20, Säule D (innen) = 30 mm L (Packung) 130 mm
eluiert mit 100 ml H₂O und 100 ml H₂O/5 % Isopropanol
Substanz kommt bereits mit der Wasserphase, im Isopropanol-Teil ist nur noch Verunreinigung.
Nach analytischer HPLC wurden 3 Fraktionen erhalten:
   F1 = 13,5 mg
   F2 = 5,5 mg
   F3 = 11,5 mg
   Gesamt = 30,5 mg = 12,2 %

Die Identität von Pyrrolidinyl-NAD (Verbindung 18) wurde durch NMR-Untersuchungen bestätigt.

### Glucosedehydrogenase-Assay für pNAD

Um die Rolle von pNAD als Cofaktor für Glucosedehydrogenase (GlucDH) zu untersuchen, wurde eine GlucoseDH-Aktivitätsassay in 0,1 m Tris/0,2 M NaCl (pH 8,5) Puffer durchgeführt. Die Konzentration Glucose betrug 80 mM. Es wurden pNAD- bzw. als NAD-Konzentrationen von 0,05-0,5 mM eingesetzt. Hierzu wurden 10 (pNAD) oder 0,002 mg/ml (NAD), [83 bzw. 0,017 µm] GlucDH hinzugegeben. Der Assay wurde bei Raumtemperatur durchgeführt, wobei die enzymatische Reaktion durch Aufnahme von Absorptionsspektren in regelmäßigen Zeitabständen verfolgt wurde. Die in Tabelle 1 angegebenen Werte beziehen sich auf eine Absorptionsmessung nach 4 min.

**Tabelle 1**

| **(p)NAD (mM)** | **U/ml** | **% Aktivität** | **[GlucDH] verwendet** |
|---|---|---|---|
| 0,05 NAD | 539 | 100 | 0,002 mg/ml |
| 0,4 NAD | 1556 | 100 | 0,002 mg/ml |
| | | | |
| 0,05 pNAD | 0,00017 | 0,00003 | 10 µl 10 mg/ml |
| 0,4 pNAD | 0,0024 | 0,00015 | 10 µl 10 mg/ml |

### Absorptionsspektren von pNAD und pNADH

Absorptionsspektren von NAD und pNAD bzw. NADH und pNADH sind in den Figuren 6A und 6B gezeigt. NAD und pNAD zeigen ein Absorptionsmaximum bei 260 nm. pNADH, d.h. pNAD nach GlucDH-Akitivitätsassay, zeigt im Vergleich zu NADH eine Rotverschiebung des Absorptionsmaximums von ca. 10 nm (Figur 6B).

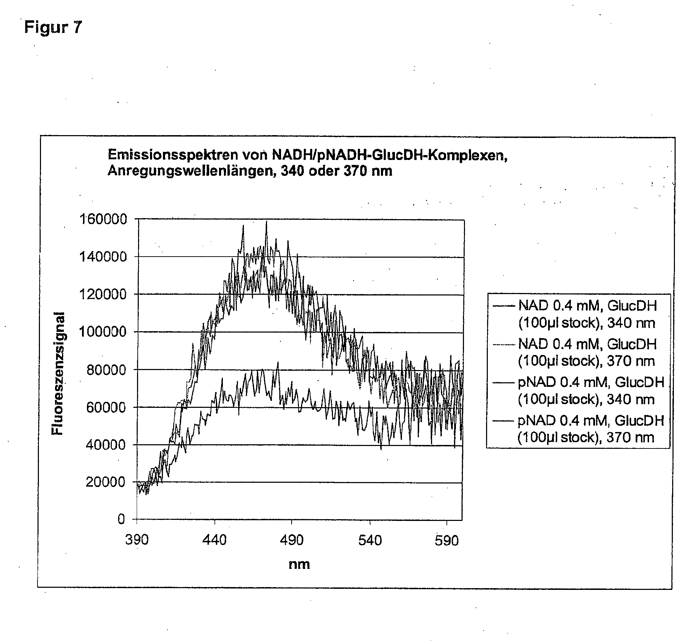

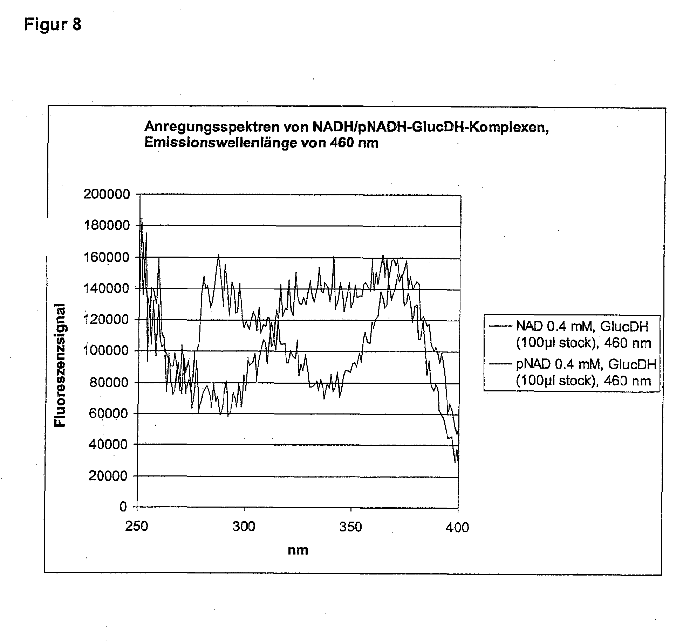

In den Figuren 7 und 8 sind ferner Fluoreszenzpektren von NADH und pNADH als GlucDH-Komplex gezeigt. Die Spektren wurden jeweils nach GlucDH-Aktivitätsassay aufgenommen. Figur 7 zeigt Emissionsspektren von NADH/pNADH-GlucDH-Komplexen bei Anregungswellenlängen von 340 oder 370 nm. Die Emissionsspektren von NADH und pNADH bei 370 nm Anregungswellenlänge sind ähnlich. Figur 8 zeigt ein Anregungsspektrum für einen NADH/pNADH-Gluc-DH-Komplex bei einer Emissionswellenlänge von 460 nm. pNADH zeigt dabei ein breiteres Anregungsspektrum als NADH. Die Spektren wurden wiederum nach GlucDH-Aktivitätsassays aufgenommen.

### Stabilitätsuntersuchung von pNAD

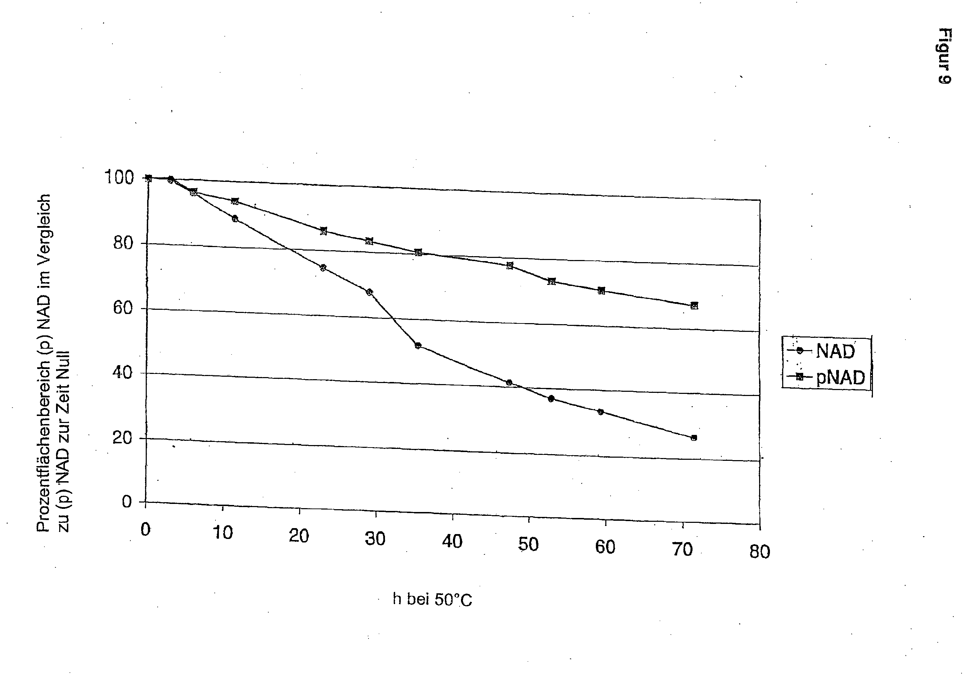

Zur Untersuchung der Stabilität von pNAD im Vergleich zu NAD wurden gleiche Mengen NAD und pNAD in jeweils 0,15 M KPO₄, 1 M NaCl-Puffer (pH 7,0) aufgenommen und bei 50 °C inkubiert. Der Zerfall von NAD bzw. pNAD wurde per HPLC verfolgt. Figur 9 zeigt den Prozentflächenbereich der (p)NAD-Mengen im Vergleich zu den (p)NAD-Mengen zur Zeit Null. Die Figur zeigt, dass pNAD im Vergleich zu NAD sehr stabil ist.

### C) Herstellung von carbaNADcyclophosphat (19)

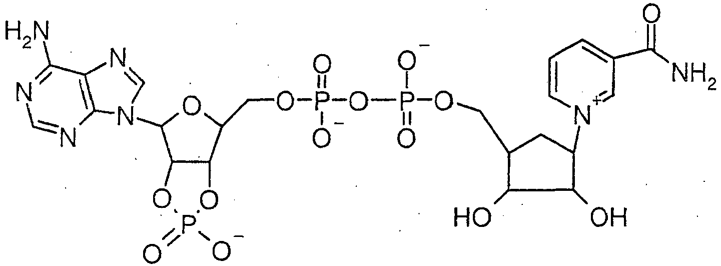

Zu 0,74 ml einer 0,2 Manganchloridlösung in absolutem Formamid wurden 79 mg (0,1 mmol) 05'-(Hydroxy-morpholino-phosphoryl)-O₂',O₃'-hydroxy-phosphoryl-adenosin, N-cyclohexyl-morpholin-4-carbonimidsäurecyclohexyl-amindsalzdihydrat (getrocknet durch Coverdampfung mit Pyridin (Morphat et al. J. Am. Chem. Soc.; 83; 1961; 663-675), 44 mg (0,105 mmol) carba-NMNmonophospaht und anschließend 25 mg trockenes Magnesiumsulfat zugegeben. Das Gemisch wurde für 3 Tage in einem geschlossenen Reaktionsgefäß unter Argon gerührt und anschließend unter Rühren in 10 ml Acetonitril eingegeben. Das Präzipitat wurde durch Filtration entfernt, durch RP-Chromatographie an einer RP 18 Hypersil ODS, 250 x 21,2 mM, 5 µm Säule unter Verwendung eines 0 % B bis 100 % B-Gradienten über 60 min gereinigt: Eluent A: 0,1 M Triethylammoniumacetat: Eluent B: 1:4-Gemisch von 0,1 M Triethylammoniumacetat und Acetonitril; Flussrate: 10 ml/min. Die Elution wurde durch Detektion bei 260 nm überwacht. Die Hauptfraktion wurde gesammelt und 5x liophylisiert, um das Triethylammoniumacetat zu entfernen. Das Triethylammoniumsalz wurde mit Dowex 50 WX2 in die freie Säure und anschließend in das Lithiumsalz umgewandelt. Ausbeute: 10 mg.

### D) Herstellung von carbaNAD (20)

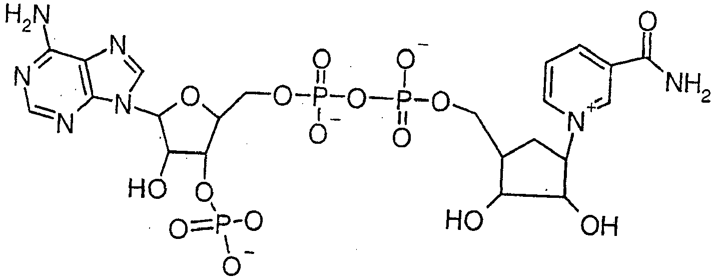

Zu einer Lösung von 2,2 mg carbaNADcyclophosphat Lithiumsalz (19) in 1 ml bis-Tris-Propanpuffer (0,02 M, pH 7,5) wurden innerhalb von 6 h dreimal vier Units Ribonuklease T2 bei 37 °C zugegeben. Das Gemisch wurde über Nacht bei 37 °C gehalten. Das Enzym wurde durch Erhitzen auf 65 °C für 20 min denaturiert. Nach Filtration wurde eine Reinigung durch RP-Chromatographie an einer RP 18 Hypersil ODS, 250 x 21,2 mm, 5 µm Säule unter Verwendung eines Gradienten von 0 % B bis 100 % B über 60 min hinweg durchgeführt: Eluent A: 0,1 M Triethylammoniumacetat; Eluent B: 1:4-Gemisch von 0,1 M Triethylammoniumacetat und Acetonitril; Flussrate: 10ml/min. Die Elution wurde durch Detektion bei 260 nm überwacht. Die Hauptfraktion wurde gesammelt und 5x liophylisiert, um das Triethylammoniumacetat zu entfernen.

Massenspektrum (MALDI Applied Biosystems Voyager System 6327: berechnet 742,45, gefunden: 743,17)

### E) Glucosedehydrogenase-Aktivitätssay für cNAD

Wie für pNAD unter B) beschrieben, wurde ein Glucosedehydrogenase-Aktivitätsassay für cNAD im Vergleich zu NAD durchgeführt. Hierzu wurden Glucose-dehydrogenasekonzentrationen von 0,1 (cNAD) oder 0,002 mg/ml (NAD), [0,83 bzw. 0,017 µm] eingesetzt. Die eingesetzten Mengen und Ergebnisse sind in Tabelle 2 gezeigt.

**Tabelle 2**

| **(c)NAD (mM)** | **U/ml** | **% Aktivität** | **[GlucDH] verwendet** |
|---|---|---|---|
| 0,05 NAD | 430 | 100 | 0,002 mg/ml |
| 0,1 NAD | 556 | 100 | 0,002 mg/ml |
| | | | |
| 0,05 cNAD | 2,7 | 0,63 | 0,1 mg/ml |
| 0,1 cNAD | 5,3 | 0,95 | 0,1 mg/ml |

### F) Absorptionsspektren von cNAD und cNADH

Die Figuren 10A, 10B bzw. 10C zeigen Absorptionsspektren von NAD und cNAD. Sowohl NAD als auch cNAD weisen bei 260 nm ein Absorptionsmaximum auf. Figur 10B zeigt Absorptionsspektren von NADH und cNADH, wobei die Spektren jeweils nach einem Glucosedehydrogenase-Aktivitätsassay aufgenommen wurden. Das Absorptionsmaximum von cNADH zeigt ein Rotverschiebung von 20 nm. Weitere Absorptionsspektren für NADH und cNADH sind in Figur 10C gezeigt, wobei wie in der Legende angegeben, unterschiedliche Bedingungen für die zugehörigen Glucosedehydrogenase-Aktivitätsassays gewählt wurden.

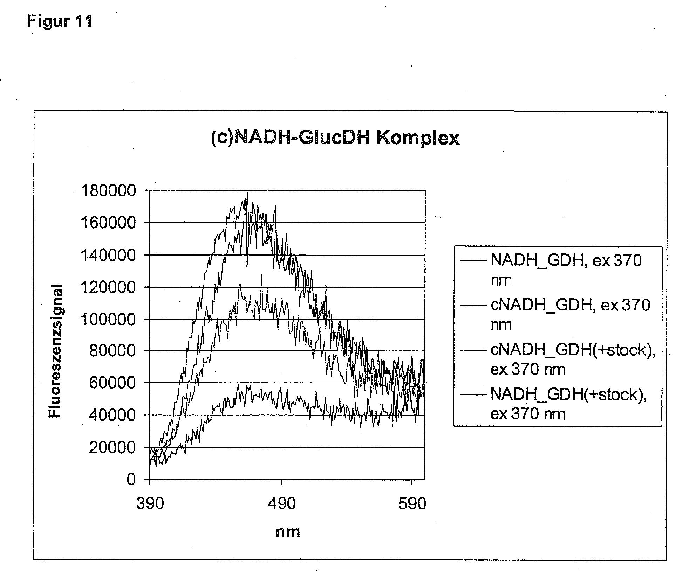

Figur 11 zeigt ferner Fluoreszenzpektren von NADH und cNADH als GlucDH-Komplex. Die Spektren wurden bei einer Anregungswellenlänge von 370 nm nach Glucosedehydrogenase-Aktivitätsassay aufgenommen. Sowohl NADH als auch cNADH zeigt bei Behandlung mit GlucDH eine Erhöhung des Fluoreszenzsignals.

## Patentansprüche

1. Testelement zur Bestimmung eines Analyten, umfassend (i) ein Coenzym abhängiges Enzym und (ii) als Coenzym eine Verbindung mit folgender allgemeiner Formel (I): 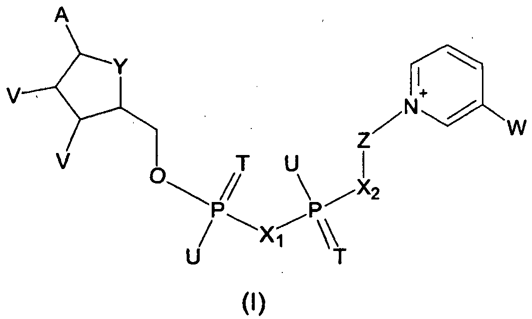 mit
A = Adenin oder ein Analog davon,
T = jeweils unabhängig O, S,
U = jeweils unabhängig OH, SH, BH₃⁻, BCNH₂⁻,
V = jeweils unabhängig OH oder eine Phosphatgruppe, wobei für den Fall, dass ein Rest V eine OH-Gruppe ist und der zweite Rest V eine Phosphatgruppe ist, die OH-Gruppe und die Phosphatgruppe mit den Kohlenstoffatomen, an welche sie gebunden sind, einen Zyklus bilden können.
W = COOR, CON(R)₂, COR, CSN(R)₂ mit R = jeweils unabhängig H oder C₁-C₂-Alkyl,
X₁, X₂ = jeweils unabhängig O, CH₂, CHCH₃, C(CH₃)₂, NH, NCH₃,
Y = NH, S, O, CH₂,
Z = ein Rest umfassend eine cyclische Gruppe mit 5 C-Atomen, die gegebenenfalls ein Heteroatom ausgewählt aus O, S und N sowie gegebenenfalls einen oder mehrere Substituenten enthält, und einen Rest CR42, wobei CR4₂ an die cyclische Gruppe und an X₂ gebunden ist, mit
R4 = jeweils unabhängig H, F, Cl, CH₃, mit der Maßgabe, dass Z und der Pyridin-Rest nicht durch eine glycosidische Bindung verknüpft sind, oder ein Salz oder gegebenenfalls eine reduzierte Form davon, wobei das Enzym eine Dehydrogenase ist, ausgewählt aus einer Lactat-Dehydrogenase (E.C.1.1.1.27, 1.1.1.28), Malat-Dehydrogenase (E.C.1.1.1.37), Glycerin-Dehydrogenase (E.C.1.1.1.6), alpha-Hydroxybutyrat-Dehydrogenase, Sorbitol-Dehydrogenase oder Aminosäure-Dehydrogenase, wie etwa L-Aminosäure-Dehydrogenase (E.C.1.4.1.5), ist.

2. Testelement nach Anspruch 1,
**dadurch gekennzeichnet,**
**dass** die Substituenten an Z ausgewählt sind aus der Grupppe F, Cl sowie C₁-C₂-Alkyl, gegebenenfalls fluoriert oder chloriert oder/und OH-substituiert, O-C₁-C₂-Alkyl.

3. Testelement nach Anspruch 1 oder 2, umfassend als Coenzym eine Verbindung mit folgender allgemeiner Formel (I'): 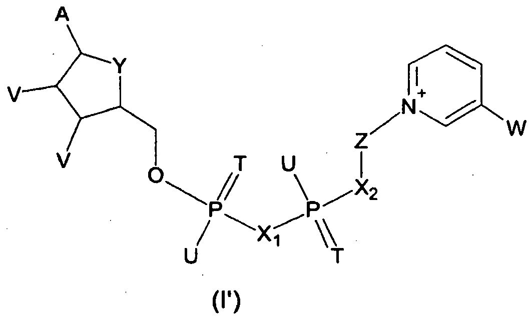 mit
A = Adenin oder ein Analog davon,
T = jeweils unabhängig O, S,
U = jeweils unabhängig OH, SH, BH₃⁻, BCNH₂⁻,
V = jeweils unabhängig OH oder eine Phosphatgruppe,
W = COOR, CON(R)₂, COR, CSN(R)₂ mit R = jeweils unabhängig H oder C₁-C₂-Alkyl,
X₁, X₂ = jeweils unabhängig O, CH₂, CHCH₃, C(CH₃)₂, NH, NCH₃,
Y = NH, S, O, CH₂,
Z = ein gesättiger oder ungesättigter carbocyclischer oder heterocyclischer Fünfring der allgemeinen Formel (II) 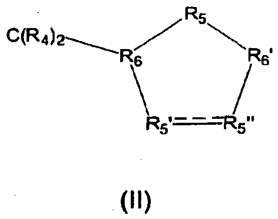 wobei zwischen R5' und R5" eine Einfach- oder Doppelbindung vorliegen kann, mit
R4 = jeweils unabhängig H, F, Cl, CH₃,
R5 = CR4₂,
wenn zwischen R5' und R5" eine Einfachbindung vorliegt, ist
R5' = O, S, NH, NC₁-C₂-Alkyl, CR4₂, CHOH, CHOCH₃,
R5" = CR4₂, CHOH, CHOCH₃,
wenn zwischen R5' und R5" eine Doppelbindung vorliegt, ist R5'=R5"= CR4,
R5'=R5"=CR4
R6, R6' = jeweils unabhängig CH, CCH₃,
oder ein Salz oder gegebenenfalls eine reduzierte Form davon.

4. Testelement nach einem der Ansprüche 1 bis 3 mit
W = CONH₂ oder COCH₃.

5. Testelement nach einem der Ansprüche 1 bis 4 in Form eines Teststreifens.

6. Verwendung des Testelements nach einem der Ansprüche 1 bis 5 zur Bestimmung von Analyten, ausgewählt aus Glucose, Milchsäure, Äpfelsäure, Glycerin, Alkohol, Cholesterin, Triglyceride, Ascorbinsäure, Cystein, Glutathion, Peptide, Harnstoff, Ammonium, Salicylat, Pyruvat, 5'-Nukleotidase, Creatinkinase (CK), Lactat-Dehydrogenase (LDH) und Kohlendioxid.

7. Verfahren zum Nachweis eines Analyten, umfassend die Schritte
(a) Inkontaktbringen einer Probe mit einem Testelement nach einem der Ansprüche 1 bis 5, umfassend ein Coenzym und
(b) Nachweisen des Analyten.

8. Verfahren nach Anspruch 7,
**dadurch gekennzeichnet,**
**dass** der Nachweis des Analyten photometrisch oder fluorometrisch erfolgt.

9. Verfahren nach Anspruch 7 oder 8,
**dadurch gekennzeichnet,**
**dass** die Probe eine Körperflüssigkeit, Abwasser oder ein Lebensmittel ist.

## Claims

1. Test element for determining an analyte comprising (i) a coenzyme-dependent enzyme and (ii) a compound of the following general formula (I) as the coenzyme: 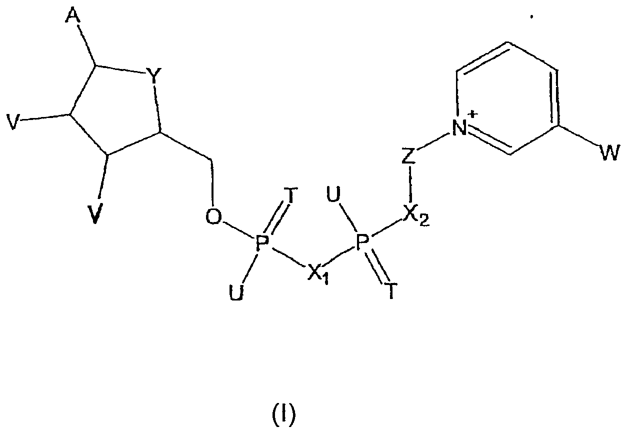 in which
A = adenine or an analogue thereof,
T = in each case independently denotes O, S,
U = in each case independently denotes OH, SH, BH₃⁻, BCNH₂⁻,
V = in each case independently denotes OH or a phosphate group, wherein in the case that a residue V is an OH group and the second residue V is a phosphate group, the OH group and the phosphate group can form a ring with the carbon atoms to which they are bound,
W = COOR, CON(R)₂, COR, CSN(R)₂ in which R in each case independently denotes H or C₁-C₂-alkyl,
X₁, X₂ = in each case independently denote O, CH₂, CHCH₃, C(CH₃)₂, NH, NCH₃,
Y = NH, S, O, CH₂,
Z = a residue comprising a cyclic group with 5 C atoms which optionally contains a heteroatom selected from O, S and N and optionally one or more substituents, and
a residue CR4₂ wherein CR4₂ is bound to the cyclic group and to X₂
where R4 = in each case independently denotes H, F, Cl, CH₃,
provided that Z and the pyridine residue are not linked by a glycosidic bond,
or a salt or optionally a reduced form thereof,
wherein the enzyme is a dehydrogenase selected from a lactate dehydrogenase (E.C.1.1.1.27, 1.1.1.28), malate dehydrogenase (E.C.1.1.1.37), glycerol dehydrogenase (E.C.1.1.1.6), alpha-hydroxybutyrate dehydrogenase, sorbitol dehydrogenase or amino acid dehydrogenase such as L-amino acid dehydrogenase (E.C.1.4.1.5)..

2. Test element according to claim 1,
**characterized in that**
the substituents on Z are selected from the group comprising F, Cl and C₁-C₂-alkyl which is optionally fluorinated or chlorinated or/and OH-substituted, O-C₁-C₂-alkyl.

3. Test element according to one of the claims 1 or 2 comprising a compound of the following general formula (I') as the coenzyme: 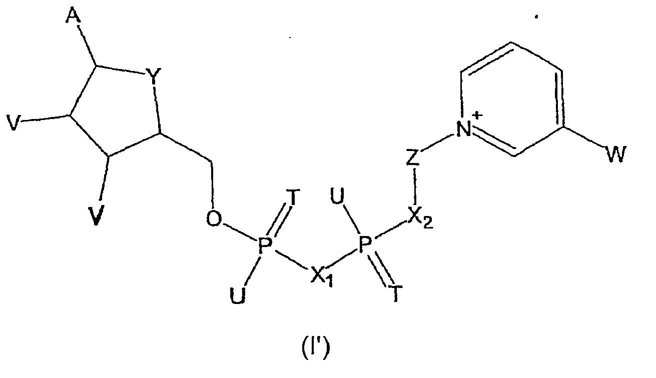 in which
A = adenine or an analogue thereof,
T = in each case independently denotes O, S,
U = in each case independently denotes OH, SH, BH₃⁻, BCNH₂⁻,
V = in each case independently denotes OH or a phosphate group,
W = COOR, CON(R)₂, COR, CSN(R)₂ in which R in each case independently denotes H or C₁-C₂-alkyl,
X₁, X₂ = in each case independently denote O, CH₂, CHCH₃, C(CH₃)₂, NH, NCH₃,
Y = NH, S, O, CH₂,
Z = a saturated or unsaturated carbocyclic or heterocyclic five-membered ring of the general formula (II) 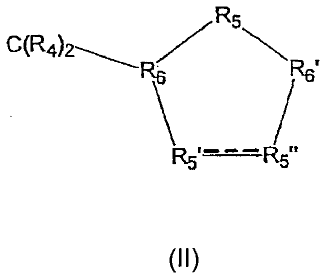 in which a single or double bond can be present between R5' and R5",
R4 = in each case independently denotes H, F, Cl, CH₃, CR4₂,
R5 = if a single bond is present between R5' and R5", then
R5' = O, S, NH, NC₁-C₂-alkyl, CR4₂, CHOH, CHOCH₃, CR4₂, CHOH, CHOCH₃,
R5" = if a double bond is present between R5' and R5", then R5' = R5" = CR4,
R6, R6' = in each case independently denote CH, CCH₃
or a salt or optionally a reduced form thereof.

4. Test element according to one of the claims 1 to 3 in which W = CONH₂ or COCH₃.

5. Test element according to one of the claims 1 to 4 in the form of a test strip.

6. Use of the test element according to one of the claims 1 to 5 to determine analytes selected from glucose, lactic acid, malic acid, glycerol, alcohol, cholesterol, triglycerides, ascorbic acid, cysteine, glutathione, peptides, urea, ammonium, salicylate, pyruvate, 5'-nucleotidase, creatine kinase (CK), lactate dehydrogenase (LDH) and carbon dioxide.

7. Method for detecting an analyte comprising the steps:
(a) contacting a sample with a test element according to one of the claims 1 to 5 comprising a coenzyme and
(b) detecting the analyte.

8. Method according to claim 7,
**characterized in that**
the analyte is detected photometrically or fluorometrically.

9. Method according to claim 7 or 8,
**characterized in that**
the sample is a body fluid, waste water or a food.

## Revendications

1. Élément de contrôle pour la détermination d'un analyte, comprenant (i) une enzyme dépendant d'une coenzyme et (ii), à titre de coenzyme, un composé répondant à la formule générale (I) suivante : 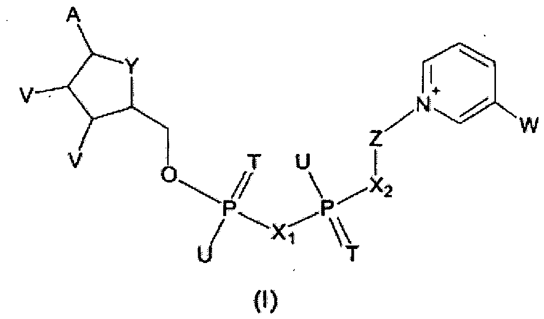 dans laquelle
A représente l'adénine ou un de ses analogues ;
T représente, chaque fois de manière indépendante, un atome d'oxygène, un atome de soufre ;
U représente, chaque fois de manière indépendante, un groupe OH, un groupe SH, un groupe BH₃⁻, un groupe BCNH₂⁻ ;
V représente, chaque fois de manière indépendante, un groupe OH ou un groupe phosphate, dans lequel, dans le cas où un résidu V représente un groupe OH et le deuxième résidu V représente un groupe phosphate, le groupe OH et le groupe phosphate peuvent former un cycle avec les atomes de carbone auxquels ils sont liés ;
W représente un groupe COOR, un groupe CON(R)₂, un groupe COR, un groupe CSN(R)₂ dans lesquels R représente, chaque fois de manière indépendante, un atome d'hydrogène ou un groupe alkyle en C₁-C₂ ;
X₁, X₂ représentent, chaque fois de manière indépendante, un atome d'oxygène, un groupe CH₂, un groupe CHCH₃, un groupe C(CH₃)₂, un groupe NH, un groupe NCH₃ ;
Y représente un groupe NH, un atome de soufre, un atome d'oxygène, un groupe CH₂ ;
Z représente un résidu comprenant un groupe cyclique contenant 5 atomes de carbone, qui contient de manière facultative un hétéroatome choisi parmi un atome d'oxygène, un atome de soufre et un atome d'azote, de même que, de manière facultative, un ou plusieurs substituants ; et
un résidu CR4₂, le résidu CR4₂ étant relié au groupe cyclique et à X₂, dans lequel R4 représente, chaque fois de manière indépendante, un atome d'hydrogène, un atome de fluor, un atome de chlore, un groupe CH₃, avec cette mesure que Z et le résidu de pyridine ne sont pas reliés par une liaison glycosydique ;
ou un de ses sels, ou de manière facultative, une de ses formes réduites, l'enzyme représentant une déshydrogénase, choisie parmi une lactate-déshydrogénase (E.C.1.1.1.27, 1.1.1.28), une malate-déshydrogénase (E.C.1.1.1.37), une glycérol-déshydrogénase (E.C.1.1.1.6), une alpha-hydroxybutyrate-déshydrogénase, une sorbitol-déshydrogénase ou une aminoacide-déshydrogénase, comme par exemple une L-aminoacide-déshydrogénase (E.C.1.4.1.5).

2. Élément de contrôle selon la revendication 1, **caractérisé en ce que** les substituants sur Z sont choisis parmi le groupe comprenant un atome de fluor, un atome de chlore et un groupe alkyle en C₁-C₂, de manière facultative fluoré ou chloré et/ou substitué avec un groupe OH, un groupe O-alkyle en C₁-C₂.

3. Élément de contrôle selon la revendication 1 ou 2, comprenant, à titre de coenzyme, un composé répondant à la formule générale (I') suivante : 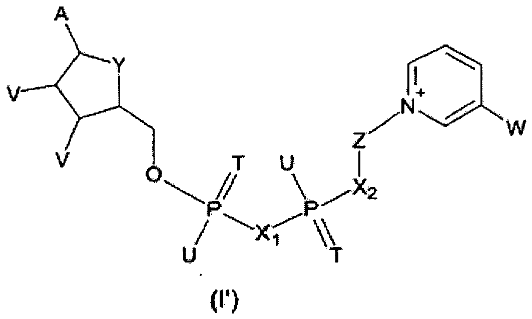 dans laquelle
A représente l'adénine ou un de ses analogues ;
T représente, chaque fois de manière indépendante, un atome d'oxygène, un atome de soufre ;
U représente, chaque fois de manière indépendante, un groupe OH, un groupe SH, un groupe BH₃⁻, un groupe BCNH₂⁻ ;
V représente, chaque fois de manière indépendante, un groupe OH ou un groupe phosphate ;
W représente un groupe COOR, un groupe CON(R)₂, un groupe COR, un groupe CSN(R)₂ dans lesquels R représente, chaque fois de manière indépendante, un atome d'hydrogène ou un groupe alkyle en C₁-C₂;
X₁, X₂ représentent, chaque fois de manière indépendante, un atome d'oxygène, un groupe CH₂, un groupe CHCH₃, un groupe C(CH₃)₂, un groupe NH, un groupe NCH₃ ;
Y représente un groupe NH, un atome de soufre, un atome d'oxygène, un groupe CH₂ ;
Z représente un noyau pentagonal carbocyclique ou hétérocyclique, saturé ou insaturé répondant à la formule générale (II) une liaison simple ou une liaison double pouvant être présente entre R5' et R5", formule dans laquelle 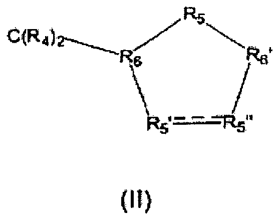
R4 représente, chaque fois de manière indépendante, un atome d'hydrogène, un atome de fluor, un atome de chlore, un groupe CH₃ ;
R5 représente un groupe CR4₂ ;
lorsqu'une liaison simple est présente entre R5' et R5",
R5' représente un atome d'oxygène, un atome de soufre, un groupe NH, un groupe N-alkyle en C₁-C₂, un groupe CR4₂, un groupe CHOH, un groupe CHOCH₃ ;
R5" représente un groupe CR4₂, un groupe CHOH, un groupe CHOCH₃ ;
lorsqu'une liaison double est présente entre R5' et R5",
R5' représente R5" qui représente CR4 ;
R6, R6' représentent, chaque fois de manière indépendante, un groupe CH, un groupe CCH₃ ;
ou un de ses sels, ou de manière facultative, une de ses formes réduites.

4. Élément de contrôle selon l'une quelconque des revendications 1 à 3, dans lequel W représente un groupe CONH₂ ou un groupe COCH₃.

5. Élément de contrôle selon l'une quelconque des revendications 1 à 4 sous la forme d'un bâtonnet diagnostique.

6. Utilisation de l'élément de contrôle selon l'une quelconque des revendications 1 à 5 pour la détermination d'analytes choisis parmi le glucose, l'acide lactique, l'acide malique, le glycérol, un alcool, le cholestérol, des triglycérides, l'acide ascorbique, la cystéine, le glutathion, des peptides, l'urée, l'ammonium, un salicylate, un pyruvate, la 5'-nucléotidase, la créatine-kinase (CK), la lactate-déshydrogénase (LDH) et le dioxyde de carbone.

7. Procédé pour le décèlement d'un analyte, comprenant les étapes dans lesquelles :
(a) on met un échantillon en contact avec un élément de contrôle selon l'une quelconque des revendications 1 à 5, comprenant une coenzyme et
(b) on décèle l'analyte.

8. Procédé selon la revendication 7, **caractérisé en ce que** le décèlement de l'analyte a lieu par voie photométrique ou par voie fluorométrique.

9. Procédé selon la revendication 7 ou 8, **caractérisé en ce que** l'échantillon est un liquide corporel, des eaux usées ou une denrée alimentaire.
